# EUROPÄISCHE PATENTSCHRIFT

(11) **EP 3 700 886 B1**
(45) Veröffentlichungstag und Bekanntmachung des Hinweises auf die Patenterteilung: **13.10.2021**
(21) Anmeldenummer: 18785387.4
(22) Anmeldetag: 17.10.2018
(51) Int. Cl.: C07C 209/84, C07C 209/86

(54) **VERFAHREN ZUR HERSTELLUNG VON ETHYLENAMINEN**
SEPARATION OF N-METHYLETHYLENDIAMINE FROM EDA-CONTAINING MIXTURES
SÉPARATION DE LA N-MÉTHYLÉTHYLÈNE DIAMINE À PARTIR DES MÉLANGES CONTENANT DE L'ÉTHYLÈNEDIAMINE

(30) Priorität: 27.10.2017 EP 17198933
(43) Veröffentlichungstag der Anmeldung: 02.09.2020
(73) Patentinhaber: BASF SE, 67056 Ludwigshafen am Rhein (DE)
(72) Erfinder: LUYKEN, Hermann, 67056 Ludwigshafen (DE)
(74) Vertreter: BASF IP Association
(86) Internationale Anmeldenummer: PCT/EP2018/078326
(87) Internationale Veröffentlichungsnummer: WO 2019/081286

(56) Entgegenhaltungen:
- EP-A1- 2 507 202
- WO-A1-2015/135971

## Beschreibung

Die vorliegende Erfindung betrifft ein Verfahren zur Aufreinigung von Ethylendiamin (EDA).

Ethylendiamin wird vorwiegend als Zwischenprodukt für die Produktion von Bleichaktivatoren, Pflanzenschutzmitteln, Pharmazeutika, Schmiermitteln, Textilharzen, Polyamiden, Papierhilfsmitteln, Benzinadditiven und vielen anderen Stoffen eingesetzt.

Zur Herstellung von EDA sind zahlreiche Verfahren bekannt (siehe beispielsweise Ullmann's Encyclopedia of Industrial Chemistry, "Amines Aliphatic", Absatz 8.1.1. DOI: 10.1002/1436007.a02_001).

Bei der Herstellung von Ethylendiamin kann N-Methylethylendiamin (NMEDA) durch Nebenreaktionen gebildet werden.

So kann zum Beispiel bei der Umsetzung von Monoethanolamin (MEA) mit Ammoniak zu EDA durch eine Abbaureaktion von Monoethanolamin direkt Kohlenmonoxid (CO) und Methylamin entstehen (Decarbonylierung) Das Methylamin kann wiederum direkt mit weiterem Monoethanolamin zu NMEDA reagieren.

NMEDA kann auch bei der Dimerisierung von Monoethanolamin zu Aminoethanolamin (AEEA entstehen, wenn AEEA direkt durch Decarbonylierung zu NMEDA abgebaut wird.

NMEDA kann sich auch bei der Herstellung von EDA aus C1-Bausteinen, wie Blausäure und Formaldehyd bilden.

Neben NMEDA können auch mehrfach N-methylierte Ethylendiamine entstehen, wie beispielsweise bis-N-Methyl-1,2-ethandiamin). Mengenmäßig ist aber üblicherweise die Bildung von NMEDA dominierend.

Für die meisten technischen Anwendungen wird vom Markt eine Reinheit für EDA von mindestens 99,5 Gew.-% gefordert. Organische Nebenkomponenten, darunter NMEDA, dürfen maximal mit einem Anteil von 0,5 Gew.-% enthalten sein. Darüber hinaus darf der Wassergehalt maximal 0,5 Gew.-% betragen.

Insbesondere wird in vielen technischen Anwendungen eine Reinheit von EDA spezifiziert, bei der Anteil an NMEDA unter 1000 Gew.-ppm liegt.

EDA, das herstellungsbedingt einen höheren NMEDA-Anteil aufweist, muss entsprechend aufgearbeitet werden, so dass EDA erhalten wird, das die geforderten Spezifikationen aufweist.

In der EP2487151 ist ein Verfahren zur Abreicherung von Alkylethylenaminen aus Ethylenaminmischungen dargestellt, wobei ein Gemisch bestehend aus Ethylendiamin, Wasser und einem oder mehreren Alkylethylendiaminen solchen Bedingungen ausgesetzt wird, dass zwischem dem Wasser und den Alkylethylenaminen ein Azeotrop gebildet wird, welches von der restlichen Zusammensetzung abgetrennt wird. Es wird offenbart, dass der Druck in der Rektifikationskolonne, in der das Azeotrop von Wasser und Alkylethylendiamin abgetrennt wird im Bereich von 1,01 bis 2,12 bar, bevorzugt 1,5 bis 1,98 bar erfolgt. In Beispiel 1 erfolgt die Destillation bei einem Kopfdruck von 1,634 bar, einer Kopftemperatur von 115°C und einer Sumpftemperatur von 176°C. Abgesehen von diesen technischen Angaben zur Destillation enthält die Offenbarung keine weiteren technischen Informationen, welche Maßnahmen der Fachmann zu treffen hat, damit sich ein Azeotrop von Alkylethylenamin und Wasser bildet.

Ein weiteres Verfahren zur Abtrennung von NMEDA von EDA ist in der EP2507202 offenbart. Diese Offenbarung lehrt, dass die Abtrennung von NMEDA in einer Rektifikationskolonne bei einem Kolonnenkopfdruck im Bereich von 0,01 bar bis 4 bar erfolgt und dass das zu destillierende Gemisch mindestens so viel Wasser enthält, dass die Bedingung H=a * X/Y erfüllt ist, wobei H der Gewichtsanteil Wasser im zu destillierenden Gemisch, X der Gewichtsanteil von Wasser und Y der Gewichtsanteil von EDA am azeotropen Punkt eines Binärgemisches von Wasser und EDA bei dem betreffenden Kolonnendruck sind und a eine reelle Zahl mit einem Wert von 0,9 und mehr ist.

Die Aufgabe der vorliegenden Erfindung bestand darin, ein Verfahren zur Aufreinigung eines EDA, welches NMEDA enthält, zur Verfügung zu stellen, so dass ein spezifikationsgerechtes EDA mit niedrigem NMEDA-Gehalt, vorzugsweise einem NMEDA-Gehalt von 1000 Gew.-ppm und weniger, erzielt werden kann. Weiterhin sollte der Energiebedarf bei der Destillation verringert werden, so dass die Aufreinigung unter wirtschaftlich günstigen Bedingungen erfolgen kann.

Die Aufgabe der vorliegenden Erfindung wurde gelöst durch ein

Verfahren zur Aufreinigung von Ethylendiamin beim dem
a) ein Gemisch enthaltend Wasser (H2O), Ethylendiamin (EDA) und N-Methylethylendiamin (NMEDA) in eine Rektifikationskolonne (NMEDA-Abtrennungskolonne) eingeleitet wird, wobei
   das eingeleitete Gemisch mindestens die Menge an Wasser enthält, wie es für die Bildung des hochsiedenden Azeotrops von EDA und Wasser bei der entsprechenden Sumpftemperatur erforderlich ist; und
b) das EDA-enthaltende Sumpfprodukt aus der NMEDA-Abtrennungskolonne in eine zweite Rektifikationskolonne (EDA-Entwässerungskolonne) eingeleitet wird,
dadurch gekennzeichnet, dass die (i) Brüden vom Kopf der EDA-Entwässerungskolonne teilweise oder vollständig in einem Kondensator kondensiert werden, der mit einem Medium gekühlt wird, welches während der Kondensation zumindest teilweise verdampft wird und der so entstehende Dampf zumindest teilweise zur Beheizung des Verdampfers der NMEDA-Abtrennungskolonne verwendet wird; und/oder (ii) die Brüden vom Kopf der EDA-Entwässerungskolonne in die NMEDA-Abtrennungskolonne eingeleitet werden.

Überraschenderweise wurde gefunden, dass die thermische Energie der Brüden der EDA-Entwässerungskolonne in geeigneter Weise zur Verringerung des Energiebedarf bei der Verdampfung in der NMEDA-Abtrennungskolonne verwendet werden können. Hierdurch konnte ein besonders energieeffizientes Aufreinigungsverfahren erschaffen werden.

Nachfolgend werden folgende Abkürzungen verwendet:
- AEEA:: Aminoethylethanolamin
- AEP:: Aminoethylpiperazin.
- DETA:: Diethylentriamin.
- EDA:: Ethylendiamin.
- EDC:: Ethylendichlorid.
- HEP:: Hydroxyethylpiperazin.
- HPA:: Heavy Polyamine (Schwere Polyamine).
- MEA:: Monoethanolamin.
- MEG:: Monoethylenglykol
- NMEDA:: N-Methylethylendiamin
- PEHA:: Pentaethylenhexamine.
- PIP:: Piperazin.
- TEPA:: Tetraethylenepentamin.
- TETA:: Triethylenetetramin.

Druckangaben beziehen sich, falls nicht anderweitig spezifiziert, auf die Angabe des absoluten Drucks.

### Gemische

In das Verfahren zur Aufreinigung von EDA werden erfindungsgemäß Gemische eingesetzt, die NMEDA, EDA und Wasser enthalten.

### Herstellung der Gemische

Die Herstellung solcher Gemische kann dadurch erfolgen, dass zunächst ein EDA-Herstellungsverfahren durchgeführt wird. Im Anschluss an das EDA- Herstellungsverfahren erfolgt in der Regel eine Ammoniakabtrennung, welche auch die Abtrennung von Wasserstoff beinhalten kann.

In der bevorzugten Ausführungsform wird der aus der Ammoniakabtrennung erhaltene Reaktionsaustrag ohne weitere Aufarbeitungsschritte direkt in das erfindungsgemäße Verfahren eingesetzt.

In einer weiteren Ausführungsform kann ein Gemisch enthaltend NMEDA. EDA und Wasser eingesetzt werden, dass dadurch erhältlich ist, dass im Anschluss an die Ammoniakabtrennung eine vollständige oder partielle Abtrennung der höher siedenden Amine (Abtrennung der höher siedenden Amine) erfolgt.

### EDA-Herstellungsverfahren

Die erste Stufe zur Herstellung der Gemische, die in das erfindungsgemäße Verfahren eingesetzt werden können, ist üblicherweise ein EDA-Herstellungsverfahren.

Die Herstellung von EDA kann durch verschiedene Verfahren erfolgen.

In einer bevorzugten Ausführungsform (MEA-Verfahren) erfolgt die Herstellung von EDA durch Umsetzung von MEA mit NH3.

In einer weiteren bevorzugten Ausführungsform (C1-Verfahren) erfolgt die Herstellung von EDA durch Umsetzung von Formaldehyd, Blausäure, Ammoniak und Wasserstoff.

In einer weiteren bevorzugten Ausführungsform (EDC-Verfahren) erfolgt die Herstellung von EDA durch Umsetzung von Ethylendichlorid mit Ammoniak (EDC-Verfahren).

In noch einer weiteren bevorzugten Ausführungsform (MEG-Verfahren) kann die Herstellung von EDA durch Umsetzung von MEG mit Ammoniak erfolgen.

### MEA-Verfahren

Die Umsetzung von MEA und Ammoniak ist beispielsweise in der US 2,861,995, der DE-A-1 172 268 und der US 3,112,318 beschrieben. Eine übersicht über die verschiedenen Verfahrensvarianten der Umsetzung von MEA mit Ammoniak kann beispielsweise dem PERP Report No. 138 "Alkyl-Amines", SRI International, 03/1981 (insbesondere Seiten 81-99, 117) entnommen werden.

Die Umsetzung von Monoethanolamin mit Ammoniak wird bevorzugt in einem Festbettreaktor an einem Übergangsmetallkatalysator bei 150-250 bar und 160-210°C oder an einem Zeolithkatalysator bei 1-20 bar und 280-380°C durchgeführt.

Bevorzugt verwendete Übergangsmetallkatalysatoren enthalten Ni, Co, Cu, Ru, Re, Rh, Pd oder Pt oder eine Mischung zweier oder mehrerer dieser Metalle auf einem oxidischen Träger (z.B. Al₂O₃, TiO₂, ZrO₂, SiO₂).

Bevorzugte Zeolithkatalysatoren sind Mordenite, Faujasite und Chabazite.

Zur Erzielung einer möglichst hohen EDA-Selektivität wird bei der übergangsmetallkatalyse im Allgemeinen mit einem molaren Verhältnis von Ammoniak zu Monoethanolamin von 6-20, bevorzugt 8-15, und bei Zeolithkatalyse von im allgemeinen 20-80, bevorzugt 30-50, gearbeitet. Der MEA-Umsatz wird in der Regel im Bereich zwischen 10 % und 80 %, bevorzugt 40-60 %, gehalten.

Im kontinuierlichen Betrieb wird bevorzugt eine Katalysatorbelastung im Bereich von 0,3-0,6 kg/(kg*h) (kg MEA pro kg Kat. pro Stunde) eingestellt.

Zur Aufrechterhaltung der Katalysatoraktivität werden bei Einsatz von Metallkatalysatoren bevorzugt zusätzlich 0,05-0,5 Gew.-% (bezogen auf den Reaktionseintrag MEA +NH₃+H₂) Wasserstoff in den Reaktor gefahren.

### C1-Verfahren

Die Herstellung eines Reaktionsaustrags kann auch durch die Umsetzung von Formaldehyd, Blausäure, Ammoniak und Wasserstoff erfolgen.

So wird in US-A 2 519 803 ein Verfahren zur Herstellung von Ethylendiamin durch die Hydrierung eines teilweise aufgereinigten wässrigen Reaktionsgemisches beschrieben, welches aus einer Aminierung von Formaldehydcyanhydrin (FACH) resultiert und als Zwischenprodukt Aminoacetonitril enthält. Formaldehydcyanhydrin kann wiederum durch Umsetzung von Formaldehyd mit Blausäure erhalten werden. Eine Verfahrensbeschreibung zur Herstellung von FACH findet sich beispielsweise in der Anmeldung PCT/EP2008/052337, Seite 26 und in der Anmeldung WO-A1-2008/104582, Seite 30 (Variante a) und b)), auf die an dieser Stelle ausdrücklich Bezug genommen wird.

DE-A 1 154 121 betrifft ein weiteres Verfahren zur Herstellung von Ethylendiamin, wobei die Edukte Blausäure, Formaldehyd, Ammoniak und Wasserstoff in Gegenwart eines Katalysators in einem so genannten Eintopf-Verfahren zur Reaktion gebracht werden.

Die WO-A1- 2008/104592 betrifft ein Verfahren zur Herstellung von EDA durch Hydrierung von Aminoacetonitril. Aminoacetonitril wird üblicherweise durch Umsetzung von Formaldehydcyanhydrin mit Ammoniak erhalten werden, wobei Formaldehydcyanhydrin wiederum in der Regel aus Blausäure und Ammoniak hergestellt wird.

Bevorzugt wird ein Reaktionsaustrag enthaltend EDA und NMEDA gemäß dem in der WO-A-2008/104592 beschriebenen Verfahren hergestellt, auf das hiermit ausdrücklich Bezug genommen wird.

### EDC-Verfahren

EDA kann auch durch Umsetzung von Ethylendichlorid mit Ammoniak (EDC-Verfahren) hergestellt werden. Die Umsetzung von EDC mit Ammoniak wird beispielsweise in der EP 2346809, in dem oben genannten PERP-Report und in den darin aufgeführten Referenzen beschrieben.

### MEG-Verfahren

In einer weiteren Ausführungsform kann EDA durch Umsetzung von MEG mit Ammoniak hergestellt werden. Die Umsetzung von MEG mit Ammoniak kann in der Flüssigphase oder der Gasphase erfolgen. Gasphasen-Umsetzungen werden beispielsweise in der CN 102190588 und CN 102233272 offenbart, während Umsetzungen in der Flüssigphase beispielsweise in der US 4,111,840, der US 3,137,730, der DE 1 72 268 und der WO 2007/093514 offenbart werden.

### Zusammensetzung der Reaktionsausträge aus den EDA-Herstellungsverfahren

Die Gemische, welche durch die oben genannten Herstellungsverfahren hergestellt werden, enthalten neben

EDA, NMEDA und Wasser, je nach Herstellungsmethode in der Regel auch noch
Wasserstoff;
Ammoniak;
höher siedende Amine;
Ethylenglykol (MEG); und
organische Nebenprodukte.

Unter höher siedenden Amine werden im Folgenden azyklische und zyklische Verbindungen bezeichnet, die 2 und mehr Amingruppen (primäre, sekundär oder tertiär) enthalten, oder die eine oder mehrere Amingruppen und ein oder mehrere OH-Gruppen enthalten, und die bei gleichem Druck einen höheren Siedepunkt als EDA aufweisen, beispielsweise Piperazin (PIP), Monoethanolamin (MEA), Diethylentriamin (DETA), Aminoethylethanolamin (AEEA), Triethylentetramin (TETA) und höhere Ethylenamine (Als höhere Ethylenamine werden im Folgenden solche höher siedenen Ethylenamine bezeichnet, welche einen höheren Siedepunkt als TETA aufweisen, z.B TEPA).

Unter organischen Nebenprodukten werden im Folgenden alle nicht umgesetzten Ausgangsprodukte und Reaktionsprodukte bezeichnet, welche keine höher siedenden Amine, MEG, Wasser, Wasserstoff, Ammoniak oder NMEDA sind.

### Ammoniakabtrennung

Die Gemische aus den oben genannten Herstellungsverfahren enthalten in der Regel Ammoniak.

Die Menge an Ammoniak in den Reaktionsausträgen liegt typischerweise in dem Bereich von 50 bis 90 Gew.-%, besonders bevorzugt im Bereich von 60 bis 85 Gew.-% und ganz besonders bevorzugt im Bereich von 70 bis 80 Gew.-%.

Bevor die Reaktionsausträge in das erfindungsgemäße Verfahren eingesetzt werden, wird üblicherweise Wasserstoff und Ammoniak aus den durch die oben genannten Herstellungsverfahren erhaltenen Gemischen abgetrennt.

Die Abtrennung von Wasserstoff und Ammoniak aus dem Reaktionsgemisch kann in für den Fachmann bekannten Verfahren erfolgen.

Vorzugsweise wird die Abtrennung von Ammoniak und Wasserstoff durch Destillation oder Rektifikation durchgeführt.

Dies kann in Destillationsblasen oder Rektifikationskolonnen erfolgen.

Im Falle einer Rektifikation können Kolonnen mit Verstärkungs- und Abtriebsteil verwendet werden.

Ist die Abreicherung von Nebenkomponenten wie Methylamin aus dem Ammoniak erforderlich, ist die Anwendung eines Verstärkungsteiles vorteilhaft.

Vorzugsweise verwendet man Kolonnen ohne Verstärkungsteil, da dann kein Rücklauf erforderlich ist, wodurch der Energiebedarf der Rektifikation verringert wird.

Die Abtrennung von Wasserstoff und Ammoniak kann in einer einzigen Stufe bei einem bestimmten Druck durchgeführt werden oder gestaffelt in einer Serie von Einrichtungen, bei denen der Druck verändert wird, um Kopf- und Sumpftemperaturen so anzupassen, dass sie praktikabel sind.

Vorzugsweise wird der Druck und die Zusammensetzung an Kopf und Sumpf so gewählt, dass die Kondensationstemperatur höher als 20°C ist, besonders bevorzugt höher als 30 °C, ganz besonders bevorzugt höher als 35 °C ist. Ist die Kondensationstemperatur in dem genannten Bereichen, dann kann die Kühlung des Kondensators mit Kühlwasser erfolgen, welches in der Regel eine Temperatur von 20-80°C, bevorzugt 30 bis 70°C und besonders bevorzugt 35-50°C aufweist.

Die Sumpftemperatur ist bevorzugt niedriger als 250 °C, besonders bevorzugt niedriger als 220 °C, ganz besonders bevorzugt niedriger als 200 °C.

Während die Einstellung des Druckes maßgeblich für die Einstellung der Temperaturen ist, werden die Temperaturen bei der Destillation auch durch Einstellung einer bestimmten Konzentration beeinflusst. So ist es möglich, die Kondensationstemperatur am Kopf dadurch zu erhöhen, dass neben Ammoniak andere höher als Ammoniak siedende Komponenten, wie beispielsweise Wasser, über Kopf mitgezogen werden. In diesem Fall ist es vorteilhaft, den Kondensator in rückvermischter Fahrweise zu betreiben (vom Fachmann "geschlossene Kondensation" genannt), so dass die Kondensation in einem engen Temperaturbereich stattfindet. Für diese Art der Kondensation ist ein Kondensator geeignet, in dem die Kondensation im Gleichstrom mit dem Abfluss des Kondensates stattfindet oder ein Direktkondensator, bei dem kalte Flüssigkeit, die umgepumpt wird, in Kontakt mit den zu kondensierenden Brüden (Dampf) gebracht wird.

Bevorzugt wird in einer ersten Stufe bei hohem Druck, beispielsweise höher als 10 bar, bevorzugt höher als 15 bar, besonders bevorzugt höher als 20 bar die Hauptmenge an Ammoniak abdestilliert, wobei im Sumpf noch eine bestimmte Ammoniakkonzentration zugelassen wird, mit der die gewünschte Sumpftemperatur eingestellt wird. Der im Reaktionsaustrag enthaltene Wasserstoff wird dabei ebenfalls über Kopf abgetrennt. Bevorzugt wird in einem ersten Kondensator dabei die Hauptmenge des Ammoniaks bei einer relativ hohen Temperatur aus den Brüden auskondensiert. Dabei wird Wasserstoff entsprechend der Taulinie des Gemisches in der Gasphase angereichert. Da ein vollständiges Kondensieren des Gemisches unter üblichen Umgebungstemperaturen nicht möglich ist, entsteht dadurch ein gasförmiger Austrag am Kondensator. Dieses kann in einen zweiten Kondensator eingeleitet werden, bei dem die Temperatur durch Kühlung mit einem kälteren Kühlmittel weiter gesenkt werden kann, so dass Ammoniak weiter aus der Gasphase abgereichert wird und ein zweites Abgas mit niedrigerem Ammoniakgehalt entsteht. Das Abgas aus dem ersten oder aus dem zweiten Kondensator kann auch durch Wäsche behandelt werden, um den darin enthaltenen Ammoniak zum größten Teil zurückzugewinnen. Dies kann durch Anwendung üblicher, dem Fachmann bekannter Verfahren, wie Waschkolonnen oder Venturiwäscher erfolgen. Dabei wird das Abgas mit einer höher als Ammoniak siedenden, bevorzugt gekühlten Flüssigkeit, bevorzugt Wasser, in Kontakt gebracht. In einer besonders bevorzugten Variante wird das Waschwasser einer anderen Stufe desselben Verfahrens entnommen. Man erhält dabei einen mit Ammoniak angereicherten flüssigen Strom und ein an Ammoniak abgereichertes Abgas, welches in der Regel abgetrennten Wasserstoff enthält. Dieses Abgas kann der Verbrennung zugeführt werden oder in ein EDA-Herstellungsverfahren zurückgeführt werdenBesonders bevorzugt wird der mit Ammoniak angereicherter Strom in die Ammoniakabtrennung, wie beispielsweise die Stufe, in die der Reaktionsaustrag eingeleitet wird, zurückgeführt.

Weiterhin bevorzugt wird der ammoniakhaltige Sumpfaustrag aus der ersten Stufe der Ammoniakabtrennung in eine zweite Stufe geleitet, die bei einem niedrigeren Druck als die erste Stufe betrieben wird. Der Druck in der zweiten Stufe wird dabei so eingestellt, dass sich die gewünschte Sumpftemperatur einstellt, wobei Ammoniak nur noch in geringer Konzentration oder überhaupt nicht im Sumpfaustrag der zweiten Stufe enthalten ist. Die Kondensationstemperatur am Kopf der zweiten Stufe wird durch Mitziehen einer höher als Ammoniak siedenden Komponente, vorzugsweise Wasser, so eingestellt, dass man mit dem gewünschten Kühlmittel, beispielsweise Flusswasser oder Umgebungsluft, das sich ergebende Gemisch kondensieren kann. In einer besonders bevorzugten Variante wird das über Kopf abgezogene, ammoniakenthaltende Gemisch in die erste Stufe zurückgeführt.

Es ist auch möglich, die Wasserstoff- und Ammoniakabtrennung in eine weitere (nullte) Stufe zu unterteilen, die der ersten Stufe vorgezogen wird und beim gleichen Druck, aber bei einer niedrigeren Sumpftemperatur wie die erste Stufe betrieben wird, so dass ein Teil vom Ammoniak bei einer niedrigeren Temperatur verdampft werden kann. Auf diese Weise kann billigere Energie bei niedrigerer Temperatur, z.B. Abfallwärme, zur Einsparung von Energiekosten verwendet werden. Vorzugsweise werden die Brüden der nullten Stufe im gleichen Kondensator wie die Brüden der ersten Stufe kondensiert.

### Zusammensetzung des Austrags aus der Ammoniakabtrennung

Nach der Abtrennung von Ammoniak und ggf. Wasserstoff wird ein Gemisch erhalten, welches neben Wasser, EDA und NMEDA in der Regel höher siedende Amine sowie organische Nebenprodukte enthält.

Nach der Abtrennung von NH3 und ggf. Wasserstoff kann der Austrag aus der Wasserstoff-Ammoniak-Abtrennung direkt in das erfindungsgemäße Verfahren eingesetzt werden.

Das nach der Ammoniakabtrennung erhaltene Gemisch, das in das erfindungsgemäße Verfahren eingesetzt werden kann, enthält bevorzugt 20 bis 75 Gew.-% EDA, besonders bevorzugt 30 bis 65 Gew.-% EDA und ganz besonders bevorzugt 35 bis 60 Gew.-% EDA.

### Das Gewichtsverhältnis von EDA zu NMEDA beträgt vorzugsweise

1 : 0,0005 (500 Gew.-ppm NMEDA) bis 1 : 0,2 (200 000 Gew.-ppm NMEDA), besonders bevorzugt 1 : 0,001 (1000 Gew.- ppm) bis 1 : 0,05 (50 000 Gew.-ppm NMEDA) und ganz besonders bevorzugt 1 : 0,005 (5000 Gew.-ppm NMEDA) bis 1 : 0,01 (10 000 Gew.-ppm NMEDA).

Der Anteil an Ammoniak beträgt bevorzugt weniger als 5 Gew.-% Ammoniak, besonders bevorzugt weniger als 2 Gew.-% Ammoniak, besonders bevorzugt weniger als 1 Gew.-% Ammoniak und insbesondere bevorzugt weniger als 0,5 Gew.-%.

Der Anteil an höher siedenden Aminen und anderen Höhersiedern wie MEG liegt bevorzugt im Bereich von 5 bis 90 Gew.-%, besonders bevorzugt im Bereich von 30 bis 85 Gew.-% und ganz besonders bevorzugt im Bereich von 40 bis 70 Gew.-%.

In einer bevorzugten Ausführungsform, beträgt das Gewichtsverhältnis der oben genannten Komponenten in dem in das Verfahren eingesetzten Gemisches vorzugsweise:
EDA : NMEDA = 1 : 0,0005 bis 0,2;
EDA : Ammoniak = 1 : 0 bis 0,05;
EDA : höher siedende Amine = 1 : 0 bis 2,0; und
EDA : organische Nebenprodukte = 1 : 0 bis 0,05.
und besonders bevorzugt:
EDA : NMEDA = 1 : 0,001 bis 0,05;
EDA : Ammoniak = 1 : 0 bis 0,025;
EDA : höher siedende Amine = 1 : 0,05 bis 1; und
EDA : organische Nebenprodukte = 1 : 0,0001 bis 0,025; und
ganz besonders bevorzugt
EDA : NMEDA = 1 : 0,005 bis 0,01;
EDA : Ammoniak = 1 : 0 bis 0,025;
EDA : höher siedende Amine = 1 : 0,05 bis 1; und
EDA : organische Nebenprodukte = 1 : 0,0001 bis 0,025.

In einer weiteren bevorzugten Ausführungsform erfolgt die Herstellung von EDA durch Umsetzung von MEG und NH3. In dieser weiteren besonders bevorzugten Ausführungsform beträgt das Gewichtsverhältnis der oben genannten Komponenten in dem in das Verfahren eingesetzten Gemisches vorzugsweise:
EDA : NMEDA = 1 : 0,0005 bis 0,2;
EDA : Ammoniak = 1 : 0 bis 0,05;
EDA : höher siedende Amine = 1 : 0 bis 2,0; und
EDA: MEG = 1 : 0,5 bis 10,0
EDA : organische Nebenprodukte = 1 : 0 bis 0,05.
und besonders bevorzugt:
EDA : NMEDA = 1 : 0,001 bis 0,05;
EDA : Ammoniak = 1 : 0 bis 0,025;
EDA : höher siedende Amine = 1 : 0,05 bis 1; und
EDA: MEG = 1 : 1,0 bis 8,0
EDA : organische Nebenprodukte = 1 : 0,0001 bis 0,025; und
ganz besonders bevorzugt
EDA : NMEDA = 1 : 0,005 bis 0,01;
EDA : Ammoniak = 1 : 0 bis 0,025;
EDA : höher siedende Amine = 1 : 0,05 bis 1; und
EDA : MEG = 1 : 2,0 bis 5,0
EDA : organische Nebenprodukte = 1 : 0,0001 bis 0,025.

Die nach der Ammoniak-Abtrennung erhaltene Gemische können direkt in das erfindungsgemäße Verfahren eingesetzt werden.

### Abtrennung der höher siedenden Amine

Alternativ zu dem direkten Einsatz des Gemisches aus der Ammoniakabtrennung kann im Anschluss an die Ammoniakabtrennung die partielle oder vollständige Abtrennung der höher siedenden Amine und anderer Hochsieder wie beispielsweise MEG erfolgen.

In einer bevorzugten Ausführungsform werden im Anschluss an die Ammoniakabtrennung alle höher siedenden Amine (inkl. PIP) abgetrennt.

Dies erfolgt bevorzugt in einer Rektifikationskolonne, die so betrieben wird, dass im unteren Teil der Kolonne, vorzugsweise im Kolonnensumpf, die höher siedenden Amine erhalten werden und im oberen Bereich der Kolonnen, vorzugsweise am Kolonnenkopf, ein Gemisch enthaltend Wasser, NMEDA und EDA abgezogen wird.

Die genauen Betriebsbedingungen der Rektifikationskolonne können entsprechend der Trennleistung der verwendeten Kolonne vom Fachmann anhand der bekannten Dampfdrucke und Verdampfungsgleichgewichte der in die Rektifikationskolonne eingeleiteten Komponenten nach herkömmlichen Berechnungsmethoden routinemäßig ermittelt werden

Am Kolonnenkopf wird ein Gemisch erhalten, das in das erfindungsgemäße Verfahren zur Abtrennung von NMEDA und EDA eingesetzt werden kann.

In einer weiteren bevorzugten Ausführungsform werden alle höher siedenden Amine und andere Höhersieder wie beispielsweise MEG, bis auf PIP, abgetrennt.

Dies erfolgt bevorzugt in einer Rektifikationskolonne, die so betrieben wird, dass im unteren Teil der Kolonne, vorzugsweise im Kolonnensumpf, die höher siedenden Amine, bis auf PIP, erhalten werden und im oberen Bereich der Kolonnen, vorzugsweise am Kolonnenkopf, ein Gemisch enthaltend Wasser, NMEDA, EDA und PIP abgezogen wird.

Die genauen Betriebsbedingungen der Rektifikationskolonne können entsprechend der Trennleistung der verwendeten Kolonne vom Fachmann anhand der bekannten Dampfdrucke und Verdampfungsgleichgewichte der in die Rektifikationskolonne eingeleiteten Komponenten nach herkömmlichen Berechnungsmethoden routinemäßig ermittelt werden

Am Kolonnenkopf wird ein Gemisch erhalten, das in das erfindungsgemäße Verfahren zur Abtrennung von NMEDA und EDA eingesetzt werden kann.

### Zusammensetzung der Gemische nach Abtrennung der höher siedenden Amine

In einer Ausführungsform in der die partielle oder vollständige Abtrennung der höher siedenden Amine und anderer Hochsieder, wie beispielsweise MEG, erfolgt, beträgt das Gewichtsverhältnis der oben genannten Komponenten in dem in das Verfahren eingesetzten Gemischs vorzugsweise:
EDA : NMEDA = 1 : 0,0005 bis 0,2;
EDA : Ammoniak = 1 : 0 bis 0,05;
EDA: PIP = 1:0 bis 0,05
EDA : höher siedende Amine = 1 : 0 bis 0,1 und
EDA : MEG = 1 : 0 bis 0,1
EDA : organische Nebenprodukte = 1 : 0 bis 0,05.
und besonders bevorzugt:
EDA : NMEDA = 1 : 0,001 bis 0,05;
EDA: PIP = 1:0 bis 0,02;
EDA : Ammoniak = 1 : 0 bis 0,025;
EDA : höher siedende Amine = 1 : 0 bis 0,05; und
EDA : MEG = 1 : 0 bis 0,05
EDA : organische Nebenprodukte = 1 : 0,0001 bis 0,025; und
ganz besonders bevorzugt
EDA : NMEDA = 1 : 0,005 bis 0,01;
EDA : Ammoniak = 1 : 0 bis 0,025;
EDA: PIP = 1 : 0 bis 0,01
EDA : höher siedende Amine = 1 : 0 bis 0,02; und
EDA : MEG = 1 : 0 bis 0,001
EDA : organische Nebenprodukte = 1 : 0,0001 bis 0,025.

In einer Ausführungsform in der die partielle oder vollständige Abtrennung der höher siedenden Amine und anderer Hochsieder, wie beispielsweise MEG bis auf PIP, erfolgt, beträgt das Gewichtsverhältnis der oben genannten Komponenten in dem in das Verfahren eingesetzten Gemischs vorzugsweise:
EDA : NMEDA = 1 : 0,0005 bis 0,2;
EDA : Ammoniak = 1 : 0 bis 0,05;
EDA: PIP = 1: 0,1 bis 2
EDA : höher siedende Amine = 1 : 0 bis 0,1 und
EDA : MEG = 1 : 0 bis 0,1
EDA : organische Nebenprodukte = 1 : 0 bis 0,05.
und besonders bevorzugt:
EDA : NMEDA = 1 : 0,001 bis 0,05;
EDA: PIP = 1: 0,2 bis 1;
EDA : Ammoniak = 1 : 0 bis 0,025;
EDA : höher siedende Amine = 1 : 0 bis 0,05; und
EDA : MEG = 1 : 0 bis 0,05
EDA : organische Nebenprodukte = 1 : 0,0001 bis 0,025; und
ganz besonders bevorzugt
EDA : NMEDA = 1 : 0,005 bis 0,01;
EDA : Ammoniak = 1 : 0 bis 0,025;
EDA: PIP = 1 : 0,3 bis 0,5
EDA : höher siedende Amine = 1 : 0 bis 0,02; und
EDA : MEG = 1 : 0 bis 0,001
EDA : organische Nebenprodukte = 1 : 0,0001 bis 0,025.

Wassermenge:
Unabhängig davon, ob das Wasser-, EDA- und NMEDA-enthaltende Gemisch direkt nach der Ammoniakabtrennung oder indirekt nach partieller oder vollständiger Abtrennung der höher siedenden Amine in das erfindungsgemäße Verfahren eingesetzt wird, muss erfindungsgemäß der Anteil von Wasser in dem Gemisch mindestens so hoch sein, wie es für die Bildung eines hochsiedenden Azeotrops mit EDA bei der entsprechenden Sumpftemperatur erforderlich ist.

In einer besonders bevorzugten Ausführungsform ist bei Vorhandensein von einer oder mehreren zusätzlichen Komponenten im dem Gemisch enthaltend EDA, NMDEA und H2O, die ein hochsiedendes Azeotrop mit Wasser bilden, zusätzlich mindestens die Wassermenge vorhanden, die der jeweiligen Konzentration der jeweiligen ein oder mehreren Komponente, die ein hochsiedendes Azeotrop mit Wasser bildet, entspricht.

Besonders bevorzugt ist ein deutlicher Überschuss an Wasser vorhanden, so dass alle hochsiedenden Azeotrope gebildet werden können, während der Überschuss an Wasser über Kopf abdestilliert wird.

### Bestimmung der azeotropen Zusammensetzung

Die Bestimmung der Zusammensetzung von Wasser und EDA am azeotropen Punkt eines Binärgemisches von Wasser und EDA in Abhängigkeit des Drucks ist dem Fachmann geläufig.

So können azeotrope Punkte in Abhängigkeit des Drucks experimentell gemessen werden, wobei die Methoden dem Fachmann bekannt sind. Azeotrope Punkte für das binäre System EDA und Wasser können beispielsweise den in dem Buch "Azeotropic Data Part I" (J. Gmehling, J. Menke, J. Krafczyk, K. Fischer, Wiley-VCH, 2004, Seite 425) aufgeführten Referenzen entnommen werden.

Zudem ist die Lage des binären Gemisches von EDA und Wasser - also eine Zusammenfassung der experimentell bestimmten Werte - ebenfalls in der einschlägigen Literatur beschrieben (siehe "Azeotropic Data Part I" von J. Gmehling, J. Menke, J. Krafczyk, K. Fischer, Wiley-VCH, 2004, Seite 425 oder "Dortmund Data Bank" (http://www.ddbst.de/new/Default.htm).

Der Gewichtsanteil von Wasser am azeotropen Punkt kann weiterhin mit Aktivitätskoeffizienten-Modellen, wie NRTL, in guter Näherung berechnet werden. Diese Methode ist standardmäßig in kommerziellen Simulationsprogrammen, wie Aspen Plus® der Fa. Aspentech implementiert. In den oben erwähnten Quellen für Gemischdaten sind auch Berechnungsparameter, z.B. NRTL-Parameter angegeben, mit deren Hilfe der azeotrope Punkt auch für von den Angaben abweichende Drücke zumindest in guter Näherung berechnet werden kann.

Bevorzugt erfolgt die Berechnung des Gewichtsanteils von Wasser am azeotropen Punkt mittels des NRTL-Modells von Aspen. Die Gasphase wird dabei in der Regel ideal gerechnet. Falls mit realer Gasphase gerechnet wird, ist das in den oben genannten Quellen für die Gemischdaten erwähnt. Die Dampfdruckkurven für EDA und Wasser können der Dortmunder Datenbank, anderen Quellen oder anderer Literatur entnommen werden. Damit lässt sich der azeotrope Punkt von EDA und Wasser in Abhängigkeit des Druckes berechnen.

Der Gewichtsanteil von Wasser am azeotropen Punkt, welcher auf Basis von Modellen, wie NRTL berechnet wird, kann innerhalb einer gewissen Fehlergrenze von der experimentell bestimmten azeotropen Zusammensetzung abweichen.

Maßgeblich für die Berechnung der zuzugebenden Menge an Wasser ist jedoch der tatsächliche, in der Realität vorkommende Gewichtsanteil von Wasser am azeotropen Punkt.

In bestimmten Fällen gibt es kein eindeutiges Azeotrop, d.h. ein eindeutiger Wert bei dem die Kurve, die auf einem Dampf-flüssig-Gleichgewichtsdiagramm eines binären Gemisches die Diagonale durchkreuzt, bei dem also die Zusammensetzung der Gasphase gleich der Zusammensetzung der Flüssigphase ist. In solchen Fällen verläuft die x,y-Kurve nahe der Diagonale und ist kaum von dieser unterscheidbar. In solchen Fällen muss die Wassermenge so hoch sein, dass die Kurve bei der entsprechenden Konzentration erkennbar oberhalb der Diagonale liegt.

### NMEDA-Abtrennung

Die Abtrennung von NMEDA (NMEDA-Abtrennung) aus den Gemischen, die wie voranstehend beschrieben bevorzugt durch ein EDA-Herstellungsverfahren, Ammoniakabtrennung und ggf. eine Abtrennung der höher siedenden Amine und ggf. MEG sowie andere Höhersieder, erhalten werden, erfolgt in einer Rektifikationskolonne (NMEDA-Abtrennungskolonne).

In der Rektifikationskolonne erfolgt im Allgemeinen eine Trennung in eine leichtsiedende Fraktion, enthaltend den wesentlichen Anteil an Wasser und NMEDA, sowie eine schwersiedende Fraktion, enthaltend den wesentlichen Anteil an EDA und ggf. die höher siedenden Amine und ggf. MEG, falls diese nicht bereits vorher von dem Gemisch teilweise oder vollständig abgetrennt worden sind.

Bevorzugt beträgt die Sumpftemperatur in der Rektifikationskolonne 155°C und weniger, ganz bevorzugt 145°C und weniger und insbesondere besonders bevorzugt 140°C und weniger. Bevorzugt liegt die Sumpftemperatur im Bereich von 50 bis 155 °C, besonders bevorzugt im Bereich von 55 bis 150°C, ganz besonders bevorzugt im Bereich von 60 bis 145°C und insbesondere bevorzugt im Bereich von 75°C bis 140°C.

Überraschenderweise kann die Konzentration von NMEDA im Austrag stark verringert werden, wenn die Sumpftemperatur im bevorzugten Bereich liegt. Dies ist umso überraschender, als NMEDA und EDA sehr ähnliche Komponenten sind, welche in der Regel nur schwer voneinander zu trennen sind.

Die für die Verdampfung erforderliche Energie wird üblicherweise durch einen Verdampfer im Kolonnensumpf eingetragen. Dabei handelt es sich bei dem Verdampfer üblicherweise um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.

Wird die Rektifikation bei einer Temperatur von 155°C und weniger durchgeführt kann der Anteil an NMEDA im Endprodukt EDA drastisch verringert werden.

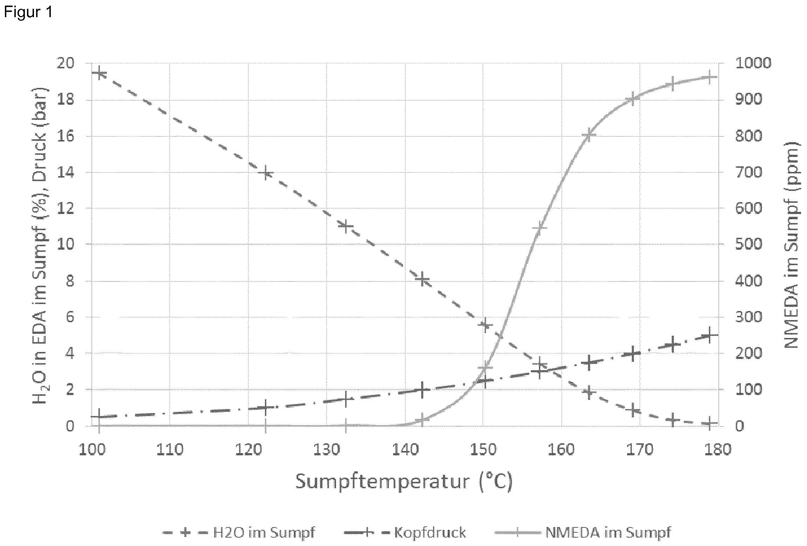

Dieser Effekt kann anhand von Figur 1 verdeutlicht werden, in dem die Menge NMEDA im Sumpf als Funktion der Sumpftemperatur aufgezeigt ist. Aus Figur 1 kann abgeleitet werden, dass die NMEDA-Menge im Sumpf oberhalb von 155°C sprunghaft ansteigt.

Die Rektifikationstemperatur in dem erfindungsgemäßen Bereich wird in der Regel durch Einstellung eines geeigneten Drucks bei der Rektifikation erreicht.

Vorzugsweise wählt man einen möglichst niedrigen Druck für die Rektifikation, und zwar besonders vorzugsweise einen solchen, bei dem eine Kondensation des am Kopf anfallenden Dampfgemisches unter technisch üblichen Bedingungen, d.h. einer Temperatur bei der noch mit Kühlwasser oder durch Umgebungsluftkühlung kondensiert werden kann. Dies sind üblicherweise Kopftemperaturen von 20 und mehr, bevorzugt 30°C und mehr und besonders bevorzugt 35°C und mehr. Bevorzugt erfolgt die Kondensation in einem Temperaturbereich von 20 bis 60 °C, bevorzugt 30 bis 55 °C, besonders bevorzugt 40 bis 50 °C.

Enthält der Eintrag in die Rektifikationskolonne im Wesentlichen keine höher siedenden Amine, ist bevorzugt am Kopf der Kolonne ein Druck von 2,5 bar und weniger, bevorzugt 1,6 bar und weniger und ganz besonders bevorzugt 1 bar und weniger einzustellen. Ein solch niedriger Druck hat zudem dem Vorteil, dass der Trennaufwand bei der Rektifikation vermindert wird. So wurde im Rahmen dieser Erfindung gefunden, dass der Trennaufwand zum Erreichen einer qualitativ gleichen Auftrennung oberhalb von 1,6 bar steil ansteigt und bei Drücken oberhalb von 2,5 bar eine gleichwertige Trennung nicht mehr möglich ist.

Enthält der Eintrag in die Rektifikationskolonne höher siedende Amine, so ist es im Allgemeinen erforderlich den Kopfdruck abzusenken, um die erfindungsgemäße Temperatur im Sumpf der Kolonne zu erreichen.

In einer besonders bevorzugten Ausführungsform beträgt der Druck am Kopf der Kolonne deshalb vorzugsweise im Bereich von 5 bis 800 mbar, besonders bevorzugt im Bereich von 10 bis 500 mbar, ganz besonders bevorzugt im Bereich von 15 bis 250 mbar und insbesondere bevorzugt 25 bis 125 mbar.

Die Rektifikation kann in dem Fachmann bekannten Apparaturen, wie Glockenboden- , Siebbodenkolonnen oder Kolonnen mit Füllkörpern oder strukturierten Packungen erfolgen. Bevorzugt werden druckverlustarme Einbauten, wie strukturierte Packungen eingesetzt, beispielsweise als Blechpackung wie Mellapak 250 Y oder Montz Pak (Typ B1-250). Es kann auch eine Packung mit geringerer oder erhöhter spezifischer Oberfläche vorliegen, oder es kann eine Gewebepackung oder eine Packung mit anderer Geometrie, wie Mellapak 252.Y, verwendet werden. Vorteilhaft bei der Verwendung dieser destillativen Einbauten sind der geringe Druckverlust und der geringe spezifische Flüssig-Hold-up im Vergleich zu beispielsweise Ventilböden. Die Einbauten können in ein oder mehreren Betten vorliegen.

Die Rektifikationskolonne enthält bevorzugt 35 bis 140 theoretische Trennstufen, besonders bevorzugt 50 bis 120 theoretische Trennstufen und ganz besonders bevorzugt 60 bis 100 theoretische Trennstufen.

Der Eintrag in die Rektifikationskolonne wird vorzugsweise in einem räumlichen Bereich zwischen 25% und 95%der theoretischen Böden der Rektifikationskolonne zugeführt (von unten gezählt), besonders bevorzugt in einem räumlichen Bereich zwischen 60% und 90% der theoretischen Böden der Rektifikationskolonne. Beispielsweise kann die Zuführung oberhalb der Mitte der theoretischen Böden erfolgen.

Die leicht siedende Fraktion, welche im Wesentlichen Wasser und NMEDA und ggf. Spuren von EDA enthält, wird vorzugsweise im oberen Bereich der Kolonne, besonders bevorzugt am Kopf der Kolonne entnommen und einem Kondensator zugeführt. Als Kondensator können beispielsweise Kondensatoren mit Kühlschlange oder Wendelrohr, Doppelrohrkühler sowie Rohrbündelwärmetauscher eingesetzt werden.

Zur Verbesserung der Abtrennung von NMEDA wird vorzugsweise das am Kondensator anfallende Kondensat zu mehr als 30 %, bevorzugt zu mehr als 50%, in den Kopf der Rektifikationskolonne zurückgeführt. Der Rest wird aus dem Verfahren ausgeschleust und in der Regel einem Sammelbehälter und von dort in im Allgemeinen einer Entsorgung, vorzugsweise einer Kläranlage, zugeführt.

Im unteren Bereich der Kolonne wird in der Regel ein Gemisch abgezogen, dass Wasser, EDA und ggf. die höher siedenden Amine sowie ggf. MEG enthält. Bevorzugt wird das Gemisch aus dem Sumpf der Kolonne abgezogen. In der Regel enthält der Sumpf mindestens soviel Wasser, wie es dem hochsiedenden Azeotrop Wasser/EDA entspricht oder etwas mehr.

### EDA-Entwässerung

Das EDA-enthaltende Sumpfprodukt aus der NMEDA-Abtrennungskolonne wird in eine zweite Rektifikationskolonne (EDA-Entwässerungskolonne) eingeleitet.

In der EDA-Entwässerungskolonne erfolgt im Allgemeinen eine Trennung in eine leichtsiedende Fraktion, enthaltend den wesentlichen Anteil an Wasser, sowie eine schwersiedende Fraktion, enthaltend den wesentlichen Anteil an EDA und ggf. die höher siedenden Amine und ggf. MEG, falls diese nicht bereits vorher von dem Gemisch teilweise oder vollständig abgetrennt worden sind.

In einer bevorzugten Ausführungsform wird der Druck in der EDA-Entwässerungskolonne so eingestellt, dass der Siedepunkt des am Kopf erhaltenen Gemisches 10°C oder höher ist als die Sumpftemperatur in der NMEDA-Abtrennungskolonne. Bevorzugt ist der Siedepunkt des am Kopf erhaltenen Gemisches 15°C oder höher, besonders bevorzugt 20°C oder höher, ganz besonders 25°C oder höher und insbesondere 30°C oder höher als die Sumpftemperatur in der NMEDA-Abtrennungskolonne. Im Rahmen der vorliegenden Erfindung ist der Siedepunkt des am Kopf der Kolonne erhaltenen Gemisches die Temperatur unterhalb der das am Kopf erhaltenen Gemisches beim jeweiligen Druck am Kopf der Kolonne flüssig vorliegt. Da es sich bei dem am Kopf der EDA-Entwässerungskolonne erhaltenen Gemisches in der Regel um ein Mehrkomponentengemisch handelt, welches neben Wasser auch noch andere Komponenten enthalten kann, ist in der Regel der Siedepunkt des am Kopf erhaltenen Gemisches nicht identisch mit dem Taupunkt des Gemisches. Im Rahmen der vorliegenden Erfindung ist der Taupunkt des Gemisches die Temperatur, an der die erste Menge Flüssigkeit aus dem am Kopf anfallenden gasförmigen Gemisches kondensiert, wenn dem Gemisch Wärme entzogen und die Temperatur verringert wird. Zwischen Taupunkt und Siedepunkt liegt in der Regel das Gemisch teilweise in der Flüssigphase und teilweise in der Gasphase vor.

Ist die Siedetemperatur des am Kopf der EDA-Entwässerungskolonne anfallenden Gemisches 10°C oder höher als die Sumpftemperatur der NMEDA-Abtrennungskolonne, ist die Temperaturdifferenz zwischen EDA-Entwässerungskolonne und NMEDA-Abtrennungskolonne ausreichend groß, um die Wärme des am Kopf der EDA-Entwässerungskolonne erhaltenen Gemisches für den Betrieb der NMEDA- Abtrennungskolonne effizient nutzen zu können.

In einer bevorzugten Ausführungsform wird der Druck in der Rektifikationskolonne so eingestellt, dass EDA und Wasser kein azeotropes Gemisch bilden. Dies ist im Allgemeinen bei einer Sumpftemperatur von 160°C und mehr, bevorzugt von 180°C und mehr und insbesondere bevorzugt von 190°C und mehr der Fall. Wenn EDA und Wasser kein azeotropes Gemisch bilden, kann eine besonders gute Trennung von EDA und Wasser erreicht werden.

Die für die Verdampfung erforderliche Energie wird üblicherweise durch einen Verdampfer im Kolonnensumpf eingetragen. Dabei handelt es sich bei dem Verdampfer üblicherweise um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.

Die Rektifikationstemperatur wird in der Regel durch Einstellung eines geeigneten Drucks bei der Rektifikation erreicht.

In der bevorzugten Ausführungsform beträgt deshalb der absolute Druck am Kopf der Rektifikationskolonne 4 bar und mehr. Bevorzugt liegt der Druck am Kopf der Rektifikationskolonne im Bereich von 4 bis 30 bar, besonders bevorzugt 6 bis 10 bar und insbesondere bevorzugt 7 bis 9 bar.

Der Zulauf erfolgt besonders bevorzugt in einem räumlichen Bereich zwischen 50% und 100% der theoretischen Böden der Rektifikationskolonne. Beispielsweise kann die Zuführung auf den Kopf der Kolonne erfolgen. Die optimale Zulaufstelle kann vom Fachmann mit den üblichen Berechnungswerkzeugen ermittelt werden.

Die Anzahl der theoretischen Trennstufen liegt im Allgemeinen im Bereich von 10 bis 80, vorzugsweise 30 bis 60.

In der ersten erfindungsgemäßen Ausführungsform weist die EDA-Entwässerungskolonne einen Kondensator auf, der in der Regel bei einer Temperatur betrieben, in dem der überwiegende Teil des Wassers bei dem entsprechenden Kopfdruck kondensiert wird.

In der Regel liegt die Betriebstemperatur des Kondensators im Bereich von 150 bis 230°C, vorzugsweise 160 bis 195°C.

Als Kondensator können beispielsweise Kondensatoren mit Kühlschlange oder Wendelrohr, Doppelrohrkühler sowie Rohrbündelwärmetauscher eingesetzt werden.

Im Kondensator fällt in der Regel ein Kondensat an, welches überwiegend Wasser enthält. Vorzugsweise wird das am Kondensator anfallende Kondensat zu mehr als 50 %, bevorzugt zu mehr als 65 %, in den Kopf der Rektifikationskolonne zurückgeführt.

Das nicht zurückgeführte Kondensat kann im Allgemeinen direkt der Entsorgung zugeführt werden, beispielsweise durch Einleiten in eine Abwasseraufbereitungsanlage.

In einer weiteren bevorzugten Ausführungsform wird das nicht in die EDA-Entwässerung zurückgeführte Kondensat in den Sumpf der NMEDA-Abtrennungskolonne eingeleitet. Dies hat den Vorteil, dass die Wassermenge in der NMEDA-Abtrennungskolonne erhöht wird, so dass die NMEDA-Abtrennungskolonne so viel Wasser enthält, wie es für die Bildung eines hochsiendenen Azeotrops von EDA und Wasser erforderlich ist.

In der zweiten erfindungsgemäßen Ausführungsform werden die am Kopf der EDA-Entwässerungskolonne abgezogenen Brüden (unter "Brüden" versteht man hier den am Kopf einer Kolonne anfallenden, in der Regel dampfförmigen Strom, bevor er einem Kondensator zugeführt wird) nicht oder nur partiell kondensiert und in die NMEDA-Abtrennungskolonne eingeleitet. Diese Ausführungsform wird untenstehend erläutert.

Am Kopf der EDA-Entwässerungskolonne wird im allgemeinen ein Gemisch erhalten, welches überwiegend Wasser enthält.

Der schwersiedende Austrag aus der EDA-Entwässerungskolonne enthält im Wesentlichen EDA und ggf. die höher siedenden Amine und ggf. MEG, wenn das EDA durch das MEG-Herstellungsverfahren hergestellt wird.

Vorzugsweise enthält der schwersiedende Austrag weniger 1,0 Gew.-% Wasser, vorzugsweise weniger als 0,6 Gew.-% und besonders bevorzugt weniger als 0,5 Gew.-% Wasser.

Dieser Austrag kann, wie nachfolgend, beschrieben durch die dem Fachmann bekannten Verfahren in seine Einzelkomponenten oder geeignete Fraktionen aufgetrennt werden, um z.B. spezifikationsgerechtes EDA zu erhalten.

### Aufreinigung der EDA-haltigen Austräge aus der EDA-Entwässerung

### Verschaltung der EDA-Entwässerungskolonne mit der NMEDA-Abtrennungskolonne

Überraschenderweise kann der Energiebedarf bei der Aufreinigung von EDA verringert werden, wenn die thermische Energie der Brüden, die am Kopf der EDA-Entwässerungskolonne abgezogen werden, genutzt wird, um einen Beitrag zur Verdampfungsenergie zu gewinnen, der in der NMEDA-Abtrennungskolonne benötigt wird.

### Die vorliegende Erfindung betrifft deshalb ein

Verfahren zur Aufreinigung von Ethylendiamin beim dem
a) ein Gemisch enthaltend Wasser (H2O), Ethylendiamin (EDA) und N-Methylethylendiamin (NMEDA) in eine Rektifikationskolonne (NMEDA-Abtrennungskolonne) eingeleitet wird, wobei
   das eingeleitete Gemisch mindestens die Menge an Wasser enthält, wie es für die Bildung des hochsiedenden Azeotrops von EDA und Wasser bei der entsprechenden Sumpftemperatur erforderlich ist; und
b) das EDA-enthaltende Sumpfprodukt aus der NMEDA-Abtrennungskolonne in eine zweite Rektifikationskolonne (EDA-Entwässerungskolonne) eingeleitet wird,
dadurch gekennzeichnet, dass die (i) Brüden vom Kopf der EDA-Entwässerungskolonne teilweise oder vollständig in einem Kondensator kondensiert werden, der mit einem Medium gekühlt wird, welches während der Kondensation zumindest teilweise verdampft wird und der so entstehende Dampf zumindest teilweise zur Beheizung des Verdampfers der NMEDA-Abtrennungskolonne verwendet wird; und/oder (ii) die Brüden vom Kopf der EDA-Entwässerungskolonne in die NMEDA-Abtrennungskolonne eingeleitet werden.

Die Berechnung der Menge Wasser in dem Gemisch, welches in die NMEDA-Abtrennungskolonne eingeleitet, wie sie für die Bildung des hochsiedenden Azeotrops von EDA und Wasser bei der entsprechenden Sumpftemperatur erforderlich ist, erfolgt, wie voranstehend beschrieben.

Bevorzugt werde die Temperatur und der Druck in der NMEDA-Abtrennungskolonne so gewählt, dass sich Druck und Temperatur in den zuvor beschriebenen bevorzugten Bereichen befinden.

Bevorzugt beträgt die Sumpftemperatur in der Rektifikationskolonne 155°C und weniger, bevorzugt 145°C und weniger und ganz besonders bevorzugt 140°C und weniger. Bevorzugt liegt die Sumpftemperatur im Bereich von 50 bis 155°C, besonders bevorzugt im Bereich von 55 bis 150°C, ganz besonders bevorzugt im Bereich von 60 bis 145°C und insbesondere bevorzugt im Bereich von 75°C bis 140°C.

Enthält der Eintrag in die Rektifikationskolonne im Wesentlichen keine höher siedenden Amine, ist bevorzugt am Kopf der Kolonne ein Druck von 2,5 bar und weniger, bevorzugt 1,6 bar und weniger und ganz besonders bevorzugt 1 bar und weniger einzustellen.

Enthält der Eintrag in die Rektifikationskolonne höher siedende Amine, so liegt der Druck am Kopf der Kolonne vorzugsweise im Bereich von 5 bis 800 mbar, besonders bevorzugt im Bereich von 10 bis 500 mbar, ganz besonders bevorzugt im Bereich von 15 bis 250 mbar und insbesondere bevorzugt 25 bis 125 mbar.

In einer bevorzugten Ausführungsform liegt die Siedetemperatur des am Kopf der EDA-Entwässerungskolonne erhaltenen Gemischs 10°C oder höher als die Sumpftemperatur in der NMEDA-Abtrennungskolonne. Bevorzugt ist der Siedepunkt des am Kopf erhaltenen Gemisches 15°C oder höher, besonders bevorzugt 20°C oder höher, ganz besonders 25°C oder höher und insbesonder 30°C oder höher als die Sumpftemperatur in der NMEDA-Abtrennungskolonne.

Im Rahmen der vorliegenden Erfindung ist der Siedepunkt des am Kopf der Kolonne erhaltenen Gemisches die Temperatur unterhalb der das am Kopf erhaltenen Gemisches beim jeweiligen Druck am Kopf der Kolonne flüssig vorliegt. Da es sich bei dem am Kopf der EDA-Entwässerungskolonne erhaltenen Gemisches in der Regel um ein Mehrkomponentengemisch handelt, welches neben Wasser auch noch andere Komponenten enthalten kann, ist in der Regel der Siedepunkt des am Kopf erhaltenen Gemisches nicht identisch mit dem Taupunkt des Gemisches. Im Rahmen der vorliegenden Erfindung ist der Taupunkt des Gemisches die Temperatur, an der die erste Menge Flüssigkeit aus dem am Kopf anfallenden gasförmigen Gemisches kondensiert, wenn dem Gemisch Wärme entzogen und die Temperatur verringert wird. Zwischen Taupunkt und Siedepunkt liegt in der Regel das Gemisch teilweise in der Flüssigphase und teilweise in der Gasphase vor.

Damit sich die entsprechende Siedetemperatur ausbildet, ist es bevorzugt die Temperatur und der Druck in der EDA-Entwässerungskolonne so zu wählen, dass sich Druck und Temperatur in den zuvor beschriebenen bevorzugten Bereichen befinden.

Dies ist im Allgemeinen bei einer Sumpftemperatur von 160°C und mehr, bevorzugt von 180°C und mehr und insbesondere bevorzugt von 190°C und mehr der Fall. In der bevorzugten Ausführungsform beträgt deshalb der absolute Druck am Kopf der Rektifikationskolonne bevorzugt 4 bar und mehr und liegt bevorzugt im Bereich von 5 bis 30 bar, besonders bevorzugt 6 bis 10 bar und insbesondere bevorzugt 7 bis 9 bar.

In der ersten Ausführungsform der erfindungsgemäßen Verfahren zur Aufreinigung von EDA werden die Brüden vom Kopf der EDA-Entwässerungskolonne teilweise oder vollständig in einem Kondensator kondensiert, der mit dem Kopf der EDA-Entwässerungskolonne verbunden ist. Die Kondensation erfolgt bevorzugt, wie voranstehend beschrieben.

Der Kondensator wird mit einem Medium gekühlt, welches bei der Kondensation der Brüden, die am Kopf der EDA-Entwässerungskolonne abgezogen werden, zumindest teilweise verdampft.

Da in der der Regel die Betriebstemperatur des Kondensators wie voranstehend beschrieben im Bereich von 150 bis 230°C, vorzugsweise 160 bis 195°C liegt, sollte das Medium entsprechend ausgewählt werden, dass es bei den entsprechenden Temperaturen und dem in den Kühlräumen des Kondensators herrschenden Druck verdampft.

Besonders bevorzugt wird als Kühlmedium Wasser verwendet, welches bei den typischen Betriebsbedingungen des Kondensators am Kopf der EDA-Entwässerungskolonne, zumindest teilweise, jedoch bevorzugt nahezu vollständig, verdampft.

Der bei der Verdampfung des Kühlmediums entstehende Dampf wird zumindest teilweise als Heizdampf zur Beheizung des Verdampfers der NMEDA-Kolonne verwendet.

Verdampfer, die bei der NMEDA-Abtrennungskolonne eingesetzt werden können, wurden bereits voranstehend beschrieben.

Im Verdampfer der NMEDA-Abtrennungskolonne gibt das dampfförmige Kühlmedium aus dem Kondensator der EDA-Entwässerungskolonne einen Teil seiner thermischen Energie an das als Zulauf zugeführte Sumpfprodukt der NMEDA-Abtrennungskolonne ab. Durch die so zugeführte Energie, kann das Sumpfprodukt, welches im Allgemeinen eine geringere Verdampfungstemperatur aufweist als das dampfförmige Kühlmedium aus der EDA-Entwässerungskolonne, zumindest teilweise verdampfen.

Dies hat den Vorteil, dass der Energiebedarf des primären Verdampfers der NMEDA-Abtrennungskolonne reduziert werden kann, wodurch Kosten eingespart werden.

In der zweiten Ausführungsform der erfindungsgemäßen Verfahren zur Aufreinigung von EDA werden die Brüden vom Kopf der EDA-Entwässerungskolonne teilweise oder vollständig in die NMEDA-Abtrennungskolonne geleitet.

In dieser Ausführungsform wird die thermische Energie der Brüden direkt genutzt, um einen Beitrag zur Verdampfungsenergie, die in der NMEDA-Abrennungskolonne benötigt wird, zu liefern. Damit die Brüden teilweise oder vollständig in die NMEDA-Abtrennungskolonne geleitet werden können, werden die Brüden nicht oder nur partiell kondensiert.

Es ist deshalb bevorzugt, dass die EDA-Entwässerungskolonne keinen Kondensator aufweist. Wenn die EDA-Entwässerungskolonne einen oder mehrere Kondensatoren aufweist, so werden diese in dieser bevorzugten Ausführungsform so betrieben, dass die Brüden nicht, oder nur partiell kondensieren. Dies kann beispielsweise dadurch erfolgen, dass der Kondensator am Kopf der EDA-Entwässerungskolonne eine Betriebstemperatur aufweist, die mindestens der Kondensationstemperatur der Brüden bei dem entsprechenden Kopfdruck entspricht. Eine solche Temperatur kann beispielsweise erreicht werden, in dem man dem Kondensator nicht oder nicht ausreichend kühlt.

Die vom Kopfbereich abgezogenen Brüden der EDA-Entwässerungskolonne werden in die NMEDA-Abtrennungskolonne geleitet.

Die Einleitung der Brüden in die NMEDA-Abtrennungskolonne umfasst das Einleiten in die Kolonne selbst, beispielsweise als Zulauf in den Abtriebsteil, vorzusweise den Sumpf der Kolonne, sowie das Einleiten in einen Verdampfer oder Wärmetauscher, der mit der NMEDA-Abtrennungskolonne verbunden ist und dessen Zweck es ist, Wärme zur Verdampfung des Sumpfproduktes in die NMEDA-Kolonne einzutragen.

In einer bevorzugten Ausführungsform dieser zweiten Ausführungsform werden die Brüden als Heizdampf in einen Sumpfverdampfer der NMEDA-Abtrennungskolonne geleitet. Im Verdampfer geben die Brüden aus der EDA-Entwässerungskolonne einen Teil ihrer thermischen Energie an das als Zulauf zugeführte Sumpfprodukt der NMEDA-Abtrennungskolonne ab. Durch die so zugeführte Energie, kann das Sumpfprodukt, welches im Allgemeinen eine geringere Verdampfungstemperatur aufweist als die Brüden aus der EDA-Entwässerungskolonne, zumindest teilweise verdampfen. Hierbei werden die als Heizdampf zugeführten Brüden im Verdampfer teilweise oder vollständig kondensiert. Das so anfallende Kondensat kann einer Entsorgung, vorzugsweise einer Abwasseraufreinigung, zugeführt werden oder in den Abtriebsteil der NMEDA-Abtrennungskolonne, vorzugsweise den Sumpf, eingeleitet werden.

In einer weiteren bevorzugten Ausführungsform dieser zweiten Ausführungsform werden die Brüden direkt als Zulauf in den Abtriebsteil der NMEDA-Abtrennungskolonne eingeleitet. Bevorzugt erfolgt der Zulauf in den Sumpf der NMEDA-Abtrennungskolonne. Da die NMEDA-Abtrennungskolonne in der Regel bei einem niedrigeren Druck als die EDA-Entwässerungskolonne betrieben wird, ist es vorteilhaft, wenn eine Drosselung der Brüden aus der EDA-Entwässerungskolonne erfolgt, bevor diese in die NMEDA-Abtrennungskolonne eingeleitet werden. Bevorzugt erfolgt die Drosselung der Brüden durch geeignete Einrichtungen, wie beispielsweise Drosselklappen oder Regelventile.

Da die Brüden aus der EDA-Entwässerungskolonne im Allgemeinen eine wesentliche höhere Temperatur aufweisen als die Sumpftemperatur der NMEDA-Abtrennungskolonne, können die Brüden zur Verdampfung der leichter siedendenden Komponenten im Kolonnensumpf der NMEDA-Abtrennungskolonne verwendet werden, indem sie wie voranstehend beschrieben entweder nicht oder nur partiell kondensiert werden und direkt in die NMEDA-Abtrennungskolonne eingeleitet werden, oder in dem sie zur Verdampfung eines Kühlmittes genutzt werden und das dampfförmige Kühlmittel als Wärmeträger verwendet wird.

Durch die thermische Nutzung der Brüden aus der EDA-Entwässerungskolonne kann der Energiebedarf des Verdampfers in der NMEDA-Abtrennungskolonne deutlich reduziert werden kann, und zwar in der Regel um die Energie, die bei der Kondensation der Brüden der EDA-Entwässerungskolonne anfallen würde. Dadurch werden Dampf- und Stromkosten zum Betreiben des Verdampfers der NMEDA-Abtrennungskolonne eingespart, sowie Kühlwasser- oder Betreibungskosten eines nicht mehr vorhandenen Kondensators der EDA-Entwässerungskolonne.

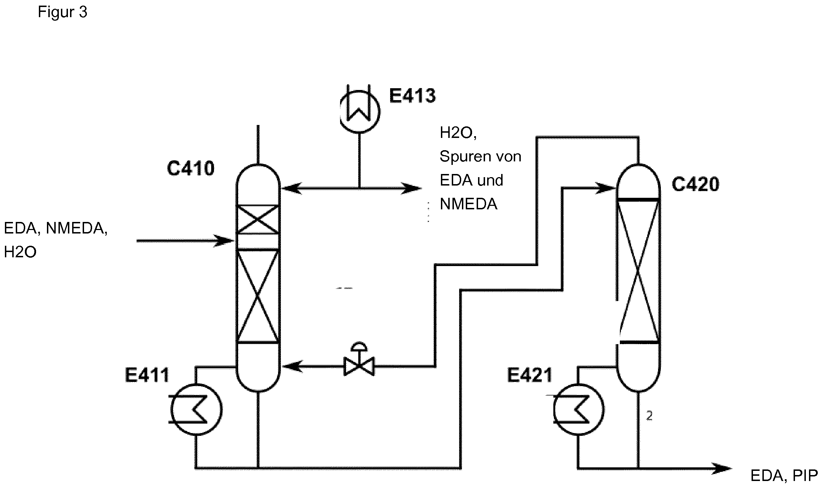

Eine bevorzugte Kolonnenverschaltung von NMEDA-Abtrennungskolonne und der EDA-Entwässerungskolonne ist in Figur 3 angegeben.

In Figur 3 wird ein Feedstrom enthaltend Wasser, NMEDA und EDA als Zulauf in den mittleren Bereich der NMEDA-Abtrennungskolonne (C410) eingeleitet. Die Kolonne C410 weist einen Verdampfer am Kolonnensumpf (E411) und einen Kondensator (E413) am Kopf der Kolonne auf.

Die NMEDA-Abtrennungskolonne wird so betrieben, dass am Kondensator E413 ein Gemisch aus Wasser, NMEDA und ggf. geringen Mengen EDA erhalten wird.

Ein Teil des Kondensatstroms wird aus dem Verfahren ausgeschleust, während der andere Teil als Rücklauf in den Kolonnenkopf der NMEDA-Abtrennungskolonne zurückgeführt wird.

Aus dem Kolonnensumpf der NMEDA-Abtrennungskolonne (C410) wird ein Gemisch enthaltend Wasser, EDA und höher siedende Amine abgezogen und in den Kopfbereich der EDA-Entwässerungskolonne (C420) geleitet.

Die EDA-Entwässerungskolonne wird so betrieben, dass am Kopf der Kolonne dampfförmige Brüden anfallen, die überwiegend Wasser enthalten und im unteren Bereich der Kolonne ein Gemisch abgezogen wird, welches EDA, PIP und ggf. höher siedende Amine enthält.

Die Brüden, die am Kopf der EDA-Entwässerungskolonne anfallen werden direkt, ohne vorherige Kondensation, in den Kolonnensumpf der NMEDA-Abtrennungskolonne (C410) geleitet. Vor der Einleitung in die NMEDA-Abtrennungskolonne werden die Brüden durch eine Drosselung, beispielsweise ein Regelventil, auf den niedrigeren Druck, der in der NMEDA-Abtrennungskolonne herrscht, gedrosselt.

Da die Brüden aus der EDA-Entwässserung eine höhere Temperatur, beispielsweise 180 bis 200°C, aufweisen als die Temperatur im Sumpf der NMEDA-Abtrennungskolonne, können die Brüden (auch nach Drosselung) die zur Verdampfung der im Kolonnensumpf befindlichen Leichtsieder erforderliche Energie mit eintragen. Dadurch kann der Energieeintrag in den Verdampfer E411 der NMEDA-Abtrennungskolonne reduziert werden.

### EDA-PIP-Abtrennung

Wenn der schwersiedende Austrag aus der EDA-Entwässerungskolonne sowohl EDA als auch höher siedende Amine enthält sowie ggf. MEG, erfolgt in der Regel zunächst eine Trennung in eine leichter siedende Fraktion, die PIP und EDA enthält, sowie eine schwersiedende Fraktion, die im Allgemeinen die höher als PIP siedenden höher siedenden Amine sowie ggf. MEG enthalten. Diese Trennung kann ebenfalls in der Rektifikationskolonne (EDA-PIP-Abtrennung) durchgeführt werden. Die genauen Betriebsbedingungen der Rektifikationskolonne können entsprechend der Trennleistung der verwendeten Kolonne vom Fachmann anhand der bekannten Dampfdrucke und Verdampfungsgleichgewichte der in die Rektifikationskolonne eingeleiteten Komponenten nach herkömmlichen Berechnungsmethoden routinemäßig ermittelt werden. Beispielsweise kann die EDA-PIP-Abtrennung so durchgeführt werden, wie in EP 2 507 202 oder in dem zuvor genannten IPRP-Report, Seite 89 ff i.V.m. Figur 6.1 offenbart, und auf die hiermit ausdrücklich Bezug genommen wird

Die schwerer siedende Fraktion wird bevorzugt am Sumpf der Rektifikationskolonne abgezogen und enthält im Allgemeinen die höher siedenden Amine.

Die höher siedenden Amine können mittels üblicher Verfahren, insbesondere durch Rektifikation in die Reinstoffe oder geeignete Fraktionen getrennt werden. Die Aufarbeitung der höher siedenden Amine ist ebenfalls im zuvor genannten PRP-Report, Seite 89 ff i.V.m. Figur 6.1 oder der EP 2487151, der EP2507202 oder der EP2346809 beschrieben.

Die leichter siedende Fraktion, die bevorzugt im oberen Bereich der Kolonnen abgezogen wird, enthält üblicherweise EDA und PIP und ist im Allgemeinen überwiegend frei von anderen höher siedenden Aminen. Der Anteil an höher siedenden Aminen (ohne Piperazin) beträgt in der Regel weniger als 0,2 Gew.-%, vorzugsweise weniger als 0,1 Gew.-% und besonders bevorzugt weniger als 0,05 Gew.-%. Um spezifikationsgerechtes EDA zu erhalten, wird die leicht siedende EDA-PIP-Fraktion in der Regel einer weiteren Aufreinigungsstufe zugeführt (EDA-Reindestillation)

### EDA-Reindestillation

Die leichtsiedende Fraktion aus der EDA/PIP-Abtrennung wird üblicherweise in eine weitere Rektifikationskolonne eingeleitet (EDA-Reindestillation), die vorzugsweise so betrieben wird, dass EDA am Kolonnenkopf anfällt und Piperazin am Kolonnensumpf abgezogen werden kann. Die genauen Betriebsbedingungen der Rektifikationskolonne können entsprechend der Trennleistung der verwendeten Kolonne vom Fachmann anhand der bekannten Dampfdrucke und Verdampfungsgleichgewichte der in die Rektifikationskolonne eingeleiteten Komponenten nach herkömmlichen Berechnungsmethoden routinemäßig ermittelt werden. Beispielsweise kann die EDA-PIP-Abtrennung so durchgeführt werden, wie in EP 2 507 202 oder im zuvor genannten PRP-Report, Seite 89 ff sowie Figur 6.1 offenbart, und auf die hiermit ausdrücklich Bezug genommen wird.

Im Kondensator fällt im Allgemeinen ein Kondensat an, welches überwiegend EDA, aber nur sehr geringe Mengen an NMEDA enthält.

Die so erhaltene leichtsiedende EDA-enthaltende Fraktion ist im Allgemeinen überwiegend frei von höher siedenden Aminen, inklusive Piperazin. Der Anteil an höher siedenden Aminen, inklusive Piperazin, beträgt in der Regel weniger als 0,5 Gew.-%, vorzugsweise weniger als 0,3 Gew.-% und besonders bevorzugt weniger als 0,2 Gew.-%.

Weiterhin enthält die leichtsiedende EDA-enthaltende Fraktion bevorzugt 99,5 Gew.-% oder mehr EDA.

Die Konzentration von NMEDA in der EDA-enthaltenden Fraktion liegt bevorzugt im Bereich von 0,001 bis 0,1 Gew.-%, bevorzugt 0,005 bis 0,08 Gew.-% und besonders bevorzugt im Bereich von 0,01 bis 0,05 Gew.-% liegt.

### Bevorzugte Kombinationen

Die voranstehend aufgeführten Verfahrensschritte und die jeweiligen Ausführungsformen der einzelnen Verfahrensschritte können in geeigneter Weise miteinander kombiniert werden, so dass die vorliegende Erfindung auch geeignete Kombinationen der voranstehend aufgeführten Verfahrensschritte und den jeweiligen Ausführungsformen umfassen.

Insbesondere sind folgende Kombination bevorzugt:
Kombination eines EDA-Herstellungsverfahren, welches ein EDA-Verfahren ist, mit einer Ammoniakabtrennung, einer NMEDA-Abtrennung, einer EDA-Entwässerung, einer EDA-PIP-Abtrennung und einer EDA-Reindestillation.
Kombination eines EDA-Herstellungsverfahren, welches ein MEG-Verfahren ist, mit einer Ammoniakabtrennung, einer NMEDA-Abtrennung, einer EDA-Entwässerung, einer EDA-PIP-Abtrennung und einer EDA-Reindestillation.
Kombination eines EDA-Herstellungsverfahren, welches ein C1-Verfahren ist, mit einer Ammoniakabtrennung, einer NMEDA-Abtrennung, einer EDA-Entwässerung, einer EDA-PIP-Abtrennung und einer EDA-Reindestillation.
Kombination eines EDA-Herstellungsverfahren, welches ein EDC-Verfahren ist, mit einer Ammoniakabtrennung, einer NMEDA-Abtrennung, einer EDA-Entwässerung, einer EDA-PIP-Abtrennung und einer EDA-Reindestillation.
Insbesondere sind solche der voranstehend genannten Kombinationen bevorzugt, in denen die jeweiligen Verfahrensschritte selber, beispielsweise die NMEDA-Abtrennung und die EDA-Entwässerung und deren energetisch vorteilhaften Verschaltungen, in den jeweiligen, bevorzugten Ausführungsformen ausgeführt werden.

### Vorteile und Anwendungen

EDA welches nach der EDA-Reindestillation oder nach der azeotropen Wasserabtrennung erhalten wurde, ist für Anwendungen vorteilhaft geeignet, bei denen es auf eine sehr hohe Reinheit des EDA ankommt.

Das so erhaltene EDA kann beispielsweise zur Herstellung von hochmolekularen Polymeren, wie Polyamiden, eingesetzt werden, da die Funktionalität des EDA nicht durch die Bildung von NMEDA verringert wird. Beispielsweise kann das so erhaltene EDA auch als Elektronikchemikalie oder als hochreine Chemikalie zur Anwendung auf dem Gebiet der Pflanzenschutzmittel, Pestizide, Epoxyharze, Komplexbildner oder für Anwendungen in der Lederindustrie, der Papierindustrie oder der Waschmittelindustrie eingesetzt werden. Die Verwendung von hochreinen Chemikalien erhöht die Ausbeute an Endprodukt, verringert die Konzentration von unerwünschten Nebenprodukten und kann weiterhin zu einer Verbesserung der Anwendungs- und Verarbeitungseigenschaften in den betreffenden Anwendungsgebieten führen. So kann NMEDA bei Polykondensationsreaktionen, beispielsweise bei der Herstellung von Epoxyharzen oder Polyamiden, zu unerwünschten Kettenabruchsreaktionen führen, welche den Polymerisationsgrad oder die Dichte an Netzwerkwerkpunkten, verringern können.

Das erfindungsgemäße Verfahren hat den Vorteil, dass der Trenn- und Energieaufwand bei der Rektifikation vermindert wird.

Mittels des erfindungsgemäßen Verfahrens zur Abtrennung von NMEDA aus einem Gemisch enthaltend EDA, NMEDA und Wasser, welches bei der Herstellung von EDA anfällt, kann zudem ein spezifikationsgerechtes EDA mit einem Gehalt von mindestens 99,5 Gew.-% EDA und einem NMEDA-Gehalt von 1000 Gew.-ppm und weniger auch dann erhalten werden, wenn sich bei der Herstellung von EDA größere Mengen an NMEDA bilden. Dies kann beispielsweise dann der Fall sein, wenn EDA aus C1-Bausteinen, wie Formaldehyd und Blausäure hergestellt wird, oder wenn Katalysatoren mit zunehmender Betriebsdauer eine partielle Deaktivierung zeigen und zur Kompensation der Deaktivierung die Reaktionstemperatur erhöht werden muss. Die Erhöhung der Temperatur hat in der Regel zufolge, dass sich die Selektivität in Bezug auf die Herstellung von EDA verschlechtert und vermehrt NMEDA als Nebenprodukt gebildet wird. Somit ermöglicht das erfindungsgemäße Verfahren auch die Erhöhung der Einsatzzeiten von Katalysatoren bei der Herstellung von EDA.

Mittels des erfindungsgemäßen Verfahrens kann somit ein hochreines EDA erhalten werden, das sich mit verbesserter Ausbeute und weniger Nebenreaktionen als Ausgangsstoff in einer Vielzahl von Anwendungen einsetzen lässt.

Das erfindungsgemäße Verfahren wird anhand von nachfolgenden Beispielen erläutert.

### Beispiel 1:

Im folgenden Beispiel wurde eine diskontinuierlich betriebene Rektifikationskolonne mit hundert theoretischen Trennstufen (versehen mit einer handelsüblichen, druckverlustarmen Packung) unter vollständigem Rückfluss (im Batch-Modus) gestellt. Die Dampfbelastung der Kolonne wurde durch Wahl eines geeigneten Durchmessers so eingestellt, dass der Druckverlust zwischen Sumpf und Kopf weniger als 5 % des eingestellten Kopfdruckes betrug, so dass der Einfluss des Druckes auf die Siedetemperatur des Gemisches entlang der Kolonne nur eine untergeordnete Rolle spielte. Durch gleichzeitige Probenahmen am Kopf und Sumpf wurde der NMEDA-und Wassergehalt bestimmt. Das Profil der Kolonne wurde durch Variation des Kolonnenprofils (Anpassung der Massen durch Anhebung oder Absenkung des Flüssigkeitsstandes im Destillatbehälter bzw. im Sumpf) so eingestellt, dass am Kopf 100 Gew.-ppm EDA vorhanden waren. Der Rest war Wasser und ggf. NMEDA. Die NMEDA-Menge im Ausgangsgemisch wurde so gewählt, dass sie 1000 Gew.-ppm bzgl. der EDA-Menge betrug. Die Wassermenge war 75 % der EDA-Menge als Masse (75 kg Wasser, 100 kg EDA). Das Ausgangsgemisch wurde im Sumpf vorgelegt, die Kolonne durch Aufheizen des Sumpfes aufgekocht unter vollständigem Rückfluss gestellt. Nach einer Wartezeit von mindestens einer Stunde wurden die Proben gezogen, so dass sichergestellt war, dass das Kolonnenprofil stationär war.

Die Ergebnisse sind in Figur 1 grafisch dargestellt.

In Figur 1 stellt die durchgezogene Kurve die NMEDA-Konzentration im Sumpf dar (auf der rechten Ordinate zu lesen) und die gestrichelte Kurve die Wasserkonzentration im Sumpf (auf der linken Ordinate zu lesen). Die Punkt-gestrichelte Kurve stellt den Kopfdruck der Kolonne dar (auf der linken Ordinate zu lesen). Die Kreuze stellen die Messpunkte dar.

Es ist deutlich zu erkennen, dass bis zu einer Sumpftemperatur von 140 °C das Gemisch im Sumpf der Kolonne praktisch NMEDA-frei ist, d.h. alles NMEDA im ursprünglichen Gemisch befindet sich mit dem restlichen Wasser am Kopf, wobei praktisch sämtliches EDA als hochsiedendes Azeotrop mit Wasser im Sumpf vorliegt (die gestrichelte Kurve stellt die Zusammensetzung des hochsiedenden Azeotropes Wasser/EDA in Abhängigkeit der Temperatur dar). Oberhalb von 140 °C beginnt eine Anreicherung von NMEDA im Sumpf, d.h. es ist weniger leicht von EDA trennbar. Bei ca. 155 °C kann nur noch die Hälfte vom NMEDA über Kopf abgetrennt werden. Oberhalb von 170 °C befindet sich der größte Teil des NMEDAs im Sumpf mit dem EDA, ist also kaum abtrennbar. Die angegebene Wasserkonzentration im Sumpf entspricht dem hochsiedenden Azeotrop Wasser/EDA bei der jeweiligen Temperatur.

Somit zeigt Beispiel 1, dass in der bevorzugten Ausführungsform der vorliegenden Erfindung, bei der die Sumpftemperatur in der NMEDA-Abtrennungskolonne 155°C und weniger beträgt eine besonders hohe EDA-Qualität erhalten werden kann.

### Beispiel 2:

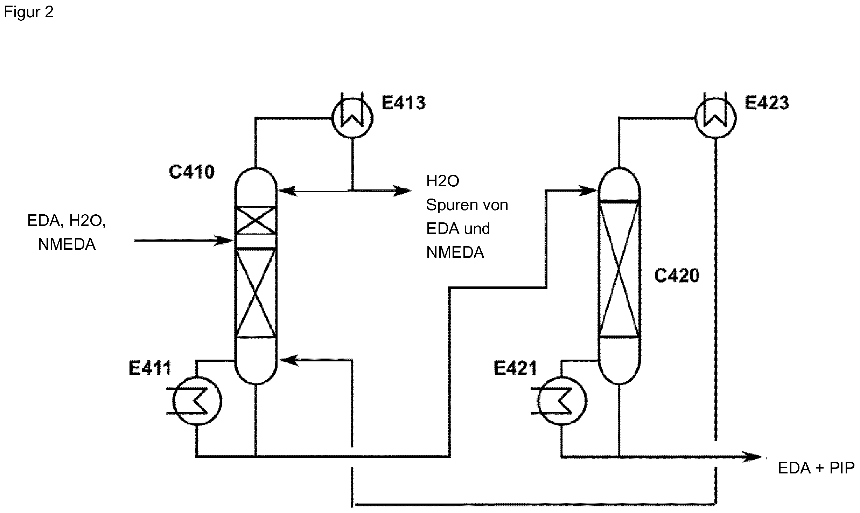

In einer Kolonnenverschaltung gemäß Figur 2 wurde ein Gemisch enthaltend 3420 kg/h Wasser, 4 kg/h NMEDA und 4160 kg/h EDA in einer ersten Kolonne bei einem Kopfdruck von 150 mbar so aufgearbeitet, dass das Produkt EDA (am Sumpf der zweiten Kolonne C420) nur noch 1 ppm NMEDA enthält. Hierzu sind ein Abtriebsteil mit 67 theoretischen Trennstufen und ein Verstärkungsteil mit 13 theoretischen Trennstufen bei einer Rücklaufmenge von 6.2 t/h erforderlich. Das über Kopf abgetrennte Wasser enthält 100 ppm EDA und praktisch das gesamte NMEDA des Zulaufgemisches. Am Verdampfer E411 müssen 4.7 MW übertragen werden. Der Sumpfaustrag der C410 wird in einer weiteren Kolonne C420 bei einem Kopfdruck von 8.5 bar so aufgearbeitet, dass das Produkt am Sumpf nur noch 0.4 % Wasser enthält. Eine weitere Verringerung des Wassergehaltes ist möglich, wenn man den Druck der Kolonne und damit die Sumpftemperatur erhöht. Die C420 enthält in diesem Beispiel nur ein Abtriebsteil. Die Brüden der C420 werden am Kondensator E423 kondensiert. Das Kondensat, das aus einem Gemisch aus EDA und Wasser besteht, wird zur C410 zurückgeführt. Der Leistungsbedarf vom Verdampfer E421 beträgt 4.1 MW. Am Kondensator E423 müssen 2.9 MW abgeführt werden.

### Beispiel 3:

In einer Kolonnenverschaltung gemäß Figur 3 erfolgte die Durchführung eines Versuchs analog Beispiel 2. Die Kolonnenverschaltung gemäß Figur 3 unterscheidet sich von der Verschaltung gemäß Figur 2 dadurch, dass der Kondensator E423 entfällt. Die Brüden der C420 werden in den Sumpf der C410 eingeleitet, ohne kondensiert zu werden. Damit ist keine Kühlleistung am Kopf der C420 erforderlich. Der Leistungsbedarf vom Verdampfer E411 geht durch die erfindungsgemäße Verschaltung von 4.7 MW auf 1.8 MW zurück, was einer deutlichen Energieeinsparung entspricht.

### Beispiel 4:

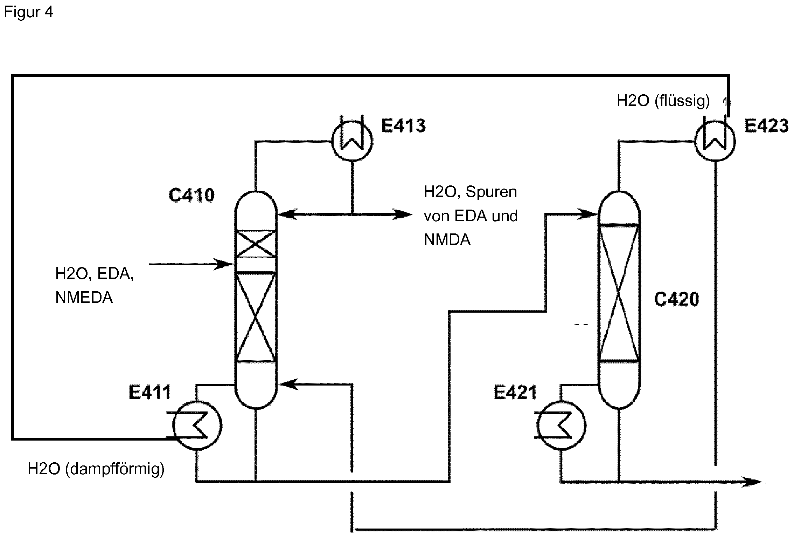

In einer Kolonnenverschaltung gemäß Figur 4 erfolgte die Durchführung eines Versuchs analog Beispiel 2. Die Kolonnenverschaltung gemäß Figur 3 unterscheidet sich von der Verschaltung gemäß Figur 2 dadurch, dass am Kondensator E423 das Kühlmittel Wasser verdampft wird und das dampfförmige Kühlmittel zur Beheizung des Verdampfers der NMEDA-Abtrennungskolonne verwendet wird.. Der Leistungsbedarf vom Verdampfer E411 kann gegenüber der Verschaltung gemäß Figur 2 erheblich verringert werden.

## Patentansprüche

1. Verfahren zur Aufreinigung von Ethylendiamin beim dem
a) ein Gemisch enthaltend Wasser (H2O), Ethylendiamin (EDA) und N-Methylethylendiamin (NMEDA) in eine Rektifikationskolonne (NMEDA-Abtrennungskolonne) eingeleitet wird, wobei
das eingeleitete Gemisch mindestens die Menge an Wasser enthält, wie es für die Bildung des hochsiedenden Azeotrops von EDA und Wasser bei der entsprechenden Sumpftemperatur erforderlich ist; und
b) das EDA-enthaltende Sumpfprodukt aus der NMEDA-Abtrennungskolonne in eine zweite Rektifikationskolonne (EDA-Entwässerungskolonne) eingeleitet wird,
**dadurch gekennzeichnet, dass** die (i) Brüden vom Kopf der EDA-Entwässerungskolonne teilweise oder vollständig in einem Kondensator kondensiert werden, der mit einem Medium gekühlt wird, welches während der Kondensation zumindest teilweise verdampft wird und der so entstehende Dampf zumindest teilweise zur Beheizung des Verdampfers der NMEDA-Abtrennungskolonne verwendet wird; und/oder (ii) die Brüden vom Kopf der EDA-Entwässerungskolonne in die NMEDA-Abtrennungskolonne eingeleitet werden.

2. Verfahrten nach Anspruch 1, **dadurch gekennzeichnet dass** der Druck am Kopf der EDA-Entwässerungskolonne so eingestellt wird, dass die Siedetemperatur des am Kopf erhaltenen Gemisches 10 °C oder höher ist als die Sumpftemperatur der NMEDA-Abtrennungskolonne.

3. Verfahren nach mindestens einem der Ansprüche 1 bis 2, **dadurch gekennzeichnet, dass** der Druck am Kopf der NMEDA-Abtrennungskolonne 2,5 bar oder weniger beträgt und der Druck am Kopf der EDA-Entwässerungskolonne 4 bar und mehr beträgt.

4. Verfahren nach mindestens einem der Ansprüche 1 bis 3, **dadurch gekennzeichnet, dass** der Druck am Kopf der NMEDA-Abtrennungskolonne im Bereich von 25 bis 200 mbar liegt.

5. Verfahren nach mindestens einem der Ansprüche 1 bis 4, **dadurch gekennzeichnet, dass** die die NMEDA-Abtrennungskolonne bei einer Sumpftemperatur T_{S} von 155°C oder weniger betrieben wird.

6. Verfahren nach mindestens einem der Ansprüche 1 bis 5, **dadurch gekennzeichnet, dass** die EDA-Entwässerungskolonne bei einer Sumpftemperatur von 180°C und mehr betrieben wird.

7. Verfahren nach mindestens einem der Ansprüche 1 bis 6, **dadurch gekennzeichnet, dass** in der Alternative (i) des Anspruchs 1 der Kondensator bei einer Temperatur im Bereich von 150-230°C betrieben wird.

8. Verfahren nach mindestens einem der Ansprüche 1 bis 7, **dadurch gekennzeichnet, dass** in der Alternative (i) des Anspruchs 1 das Kühlmedium Wasser ist.

9. Verfahren nach mindestens einem der Ansprüche 1 bis 6, **dadurch gekennzeichnet, dass** die in die NMEDA-Abtrennungskolonne eingeleiteten Brüden vom Kopf der EDA-Entwässerungskolonne nicht oder nur partiell kondensiert werden.

10. Verfahren nach Anspruch 9, **dadurch gekennzeichnet, dass** die EDA-Entwässerungskolonne keinen Kondensator aufweist oder der Kondensator am Kopf der EDA-Entwässerungskolonne so betrieben wird, dass das die Brüden nicht, oder nur partiell kondensieren.

11. Verfahren nach mindestens einem der Ansprüche 9 bis 10, **dadurch gekennzeichnet, dass** die Brüden aus der EDA-Entwässerungskolonne als Heizdampf in einen Sumpfverdampfer der NMEDA-Abtrennungskolonne eingeleitet werden.

12. Verfahren nach mindestens einem der Ansprüche 9 bis 10, **dadurch gekennzeichnet, dass** die Brüden aus der EDA-Entwässerungskolonne in den Abtriebsteil der NMEDA-Abtrennungskolonne eingeleitet werden.

13. Verfahren nach mindestens einem der Ansprüche 9 bis 10, **dadurch gekennzeichnet, dass** die Brüden aus der EDA-Entwässerungskolonne in den Sumpf der NMEDA-Abtrennungskolonne eingeleitet werden.

14. Verfahren nach mindestens einem der Ansprüche 12 bis 13, **dadurch gekennzeichnet, dass** die Brüden aus der EDA-Entwässerungskolonne vor der Einleitung in die NMEDA-Abtrennungskolonne gedrosselt werden, in dem die Brüden durch ein Expansionsventil oder Drosselklappen geleitet werden.

15. Verfahren nach mindestens einem der Ansprüche 1 bis 14, **dadurch gekennzeichnet, dass** das Sumpfprodukt der EDA-Entwässerungskolonne einer Aufarbeitung zugeführt wird, in dem zunächst EDA und PIP in einer Rektifikationskolonne von den höhersiedenden Aminen abgetrennt werden und einer weiteren Rektifikationskolonne EDA von PIP abgetrennt wird.

## Claims

1. A process for purifying ethylenediamine, in which
a) a mixture comprising water (H2O), ethylenediamine (EDA) and N-methylethylenediamine (NMEDA) is introduced into a rectification column (NMEDA removal column), where
the mixture introduced comprises at least the amount of water as required for the formation of the high-boiling azeotrope of EDA and water at the appropriate bottom temperature; and
b) the EDA-comprising bottom product from the NMEDA removal column is introduced into a second rectification column (EDA dewatering column),
which comprises (i) partly or fully condensing the vapors from the top of the EDA dewatering column in a condenser which is cooled with a medium which is at least partly evaporated during the condensation and the vapor thus formed is used at least partly to heat the evaporator of the NMEDA removal column; and/or (ii) introducing the vapors from the top of the EDA dewatering column into the NMEDA removal column.

2. The process according to claim 1, wherein the pressure at the top of the EDA dewatering column is adjusted such that the boiling temperature of the mixture obtained at the top is 10°C or higher than the bottom temperature of the NMEDA removal column.

3. The process according to at least one of claims 1 and 2, wherein the pressure at the top of the NMEDA removal column is 2.5 bar or less and the pressure at the top of the EDA dewatering column is 4 bar or more.

4. The process according to at least one of claims 1 to 3, wherein the pressure at the top of the NMEDA removal column is in the range from 25 to 200 mbar.

5. The process according to at least one of claims 1 to 4, wherein the NMEDA removal column is operated at a bottom temperature T_{S} of 155°C or less.

6. The process according to at least one of claims 1 to 5, wherein the EDA dewatering column is operated at a bottom temperature of 180°C or more.

7. The process according to at least one of claims 1 to 6, wherein, in alternative (i) of claim 1, the condenser is operated at a temperature in the range of 150-230°C.

8. The process according to at least one of claims 1 to 7, wherein, in alternative (i) of claim 1, the cooling medium is water.

9. The process according to at least one of claims 1 to 6, wherein the vapors introduced into the NMEDA removal column from the top of the EDA dewatering column are only partially condensed, if at all.

10. The process according to claim 9, wherein the EDA dewatering column does not have a condenser or the condenser at the top of the EDA dewatering column is operated such that the vapors only partially condense, if at all.

11. The process according to at least one of claims 9 and 10, wherein the vapors from the EDA dewatering column are introduced as heating vapor into a reboiler of the NMEDA removal column.

12. The process according to at least one of claims 9 and 10, wherein the vapors from the EDA dewatering column are introduced into the stripping section of the NMEDA removal column.

13. The process according to at least one of claims 9 and 10, wherein the vapors from the EDA dewatering column are introduced into the bottom of the NMEDA removal column.

14. The process according to at least one of claims 12 and 13, wherein the vapors from the EDA dewatering column are throttled before being introduced into the NMEDA removal column in that the vapors are guided through an expansion valve or throttle valves.

15. The process according to at least one of claims 1 to 14, wherein the bottom product from the EDA dewatering column is sent to a workup in which EDA and PIP are first separated from the higher-boiling amines in a rectification column and in a further rectification column EDA is separated from PIP.

## Revendications

1. Procédé pour la purification d'éthylènediamine, dans lequel
a) un mélange contenant de l'eau (H₂O), de l'éthylènediamine (EDA) et de la N-méthyléthylène-diamine (NMEDA) est envoyé dans une colonne de rectification (colonne de séparation de NMEDA),
le mélange envoyé contenant au moins la quantité d'eau qui est nécessaire pour la formation de l'azéotrope à haut point d'ébullition d'EDA et eau à la température de bas de colonne correspondante, et
b) le produit de bas de colonne, contenant de l'EDA, provenant de la colonne de séparation de NMEDA, est envoyé dans une deuxième colonne de rectification (colonne de déshydratation d'EDA),
**caractérisé en ce que** (i) les vapeurs de la tête de la colonne de déshydratation d'EDA sont partiellement ou totalement condensées dans un condenseur qui est refroidi avec un fluide qui est au moins en partie vaporisé pendant la condensation et la vapeur qui en résulte est au moins en partie utilisée pour le chauffage du rebouilleur de la colonne de séparation de NMEDA ; et/ou (ii) les vapeurs de la tête de la colonne de déshydratation d'EDA sont envoyées dans la colonne de séparation de NMEDA.

2. Procédé selon la revendication 1, **caractérisé en ce que** la pression à la tête de la colonne de déshydratation d'EDA est réglée de telle façon que la température d'ébullition du mélange obtenu à la tête est supérieure de 10 °C ou plus à la température du bas de la colonne de séparation de NMEDA.

3. Procédé selon au moins l'une quelconque des revendications 1 et 2, **caractérisé en ce que** la pression à la tête de la colonne de séparation de NMEDA est de 2,5 bars ou moins et la pression à la tête de la colonne de déshydratation d'EDA est de 4 bars et plus.

4. Procédé selon au moins l'une quelconque des revendications 1 à 3, **caractérisé en ce que** la pression à la tête de la colonne de séparation de NMEDA se situe dans la plage de 25 à 200 mbars.

5. Procédé selon au moins l'une quelconque des revendications 1 à 4, **caractérisé en ce que** la colonne de séparation de NMEDA est conduite à une température de bas de colonne Tₛ de 155 °C ou moins.

6. Procédé selon au moins l'une quelconque des revendications 1 à 5, **caractérisé en ce que** la colonne de déshydratation d'EDA est conduite à une température de bas de colonne de 180 °C et plus.

7. Procédé selon au moins l'une quelconque des revendications 1 à 6, **caractérisé en ce que** dans l'option (i) de la revendication 1 le condenseur est conduit à une température dans la plage de 150-230 °C.

8. Procédé selon au moins l'une quelconque des revendications 1 à 7, **caractérisé en ce que** dans l'option (i) de la revendication 1 le fluide de refroidissement est l'eau.

9. Procédé selon au moins l'une quelconque des revendications 1 à 6, **caractérisé en ce que** les vapeurs de la tête de la colonne de déshydratation d'EDA, envoyées dans la colonne de séparation de NMEDA, ne sont pas ou ne sont que partiellement condensées.

10. Procédé selon la revendication 9, **caractérisé en ce que** la colonne de déshydratation d'EDA ne comporte pas de condenseur ou le condenseur à la tête de la colonne de déshydratation d'EDA est conduit de telle façon que les vapeurs ne sont pas ou ne sont que partiellement condensées.

11. Procédé selon au moins l'une quelconque des revendications 9 et 10, **caractérisé en ce que** les vapeurs provenant de la colonne de déshydratation d'EDA sont envoyées en tant que vapeur surchauffée dans un rebouilleur de bas de la colonne de séparation de NMEDA.

12. Procédé selon au moins l'une quelconque des revendications 9 et 10, **caractérisé en ce que** les vapeurs provenant de la colonne de déshydratation d'EDA sont envoyées dans la section épuisement de la colonne de séparation de NMEDA.

13. Procédé selon au moins l'une quelconque des revendications 9 et 10, **caractérisé en ce que** les vapeurs provenant de la colonne de déshydratation d'EDA sont envoyées dans le bas de la colonne de séparation de NMEDA.

14. Procédé selon au moins l'une quelconque des revendications 12 et 13, **caractérisé en ce que** les vapeurs provenant de la colonne de déshydratation d'EDA sont retenues avant l'introduction dans la colonne de séparation de NMEDA, par le fait que les vapeurs sont envoyées à travers une soupape de détente ou des clapets d'étranglement.

15. Procédé selon au moins l'une quelconque des revendications 1 à 14, **caractérisé en ce que** le produit de bas de la colonne de déshydratation d'EDA est envoyé à un traitement final dans lequel d'abord l'EDA et la PIP sont séparées d'avec les aminés à plus haut points d'ébullition dans une colonne de rectification, puis l'EDA est séparée de la PIP dans une autre colonne de rectification.
